# EUROPÄISCHE PATENTSCHRIFT

(11) **EP 1 224 170 B1**
(45) Veröffentlichungstag und Bekanntmachung des Hinweises auf die Patenterteilung: **12.08.2009**
(21) Anmeldenummer: 00971347.0
(22) Anmeldetag: 09.10.2000
(51) Int. Cl.: C07D 209/34, C07D 401/12, C07D 403/12, C07D 405/12, A61K 31/404

(54) **IN 6-STELLUNG SUBSTITUIERTE INDOLINONE, IHRE HERSTELLUNG UND IHRE VERWENDUNG ALS ARZNEIMITTEL**
6-POSITION SUBSTITUTED INDOLINE, PRODUCTION AND USE THEREOF AS A MEDICAMENT
INDOLINONES SUBSTITUEES EN POSITION 6, LEUR PREPARATION ET LEUR UTILISATION EN TANT QUE MEDICAMENTS

(30) Priorität: 13.10.1999 DE 19949208; 31.08.2000 DE 10042696
(43) Veröffentlichungstag der Anmeldung: 24.07.2002
(60) Teilanmeldung: 09164507.7
(73) Patentinhaber: Boehringer Ingelheim Pharma GmbH & Co.KG, 55218 Ingelheim am Rhein (DE)
(72) Erfinder: HECKEL, Armin, 88400 Biberach (DE); ROTH, Gerald, Jürgen, 88400 Biberach (DE); WALTER, Rainer, 88400 Biberach (DE); VAN MEEL, Jacobus, A-2340 Mödling (AT); REDEMANN, Norbert, 88400 Biberach (DE); TONTSCH-GRUNT, Ulrike, A-2500 Baden (AT); SPEVAK, Walter, A-2105 Oberrohrbach (AT); HILBERG, Frank, A-1050 Wien (AT)
(86) Internationale Anmeldenummer: PCT/EP2000/009867
(87) Internationale Veröffentlichungsnummer: WO 2001/027081

(56) Entgegenhaltungen:
- WO-A-00/56710
- WO-A-99/15500
- WO-A-99/52869
- WO-A-99/62882
- DE-A- 19 815 020
- US-A- 5 886 020

## Beschreibung

Gegenstand der vorliegenden Erfindung ist die Verbindung 3-z-[1-(4-(N-((4-Methyl-piperazin-1-yl)-methylcarbonyl)-N-methyl-amino)-anilino)-1-phenyl-methylene]-6-methoxycarbonyl-2-indolinon, deren Tautomere und deren Salze.

Gegenstand der vorliegenden Erfindung sind auch physiologisch verträgliche Salze der Verbindung 3-Z-[1-(4-(N-((4-Methyl-piperazin-1-yl)-methylcarbonyl)-N-methylamino)-anilino)-1-phenyl-methylene]-6-methoxycarbonyl-2-indolinon.

Gegenstand der vorliegenden Erfindung ist auch ein Arzneimittel, enthaltend die Verbindung 3-Z-[1-(4-(N-((4-Methyl-piperazin-1-yl)-methylcarbonyl)-N-methyl-amino)-anilino)-1-phenyl-methylene]-6-methoxycarbonyl-2-indolinon oder deren Salze neben gegebenenfalls einem oder mehreren inerten Trägerstoffen und/oder Verdünnungsmitteln.

Gegenstand der vorliegenden Erfindung ist auch die Verbindung 3-Z-[1-(4-(N-((4-Methyl-piperazin-1-yl)-methylcarbonyl)-N-methyl-amino)-anilino)-1-phenyl-methylene]-6-methoxycarbonyl-2-indolinon oder deren Salze zur Verwendung als Arzneimittel.

Gegenstand der vorliegenden Erfindung ist auch die Verbindung 3-Z-[1-(4-(N-((4-Methyl-piperazin-1-yl)-methylcarbonyl)-N-methyl-amino)-anilino)-1-phenyl-methylene]-6-methoxycarbonyl-2-indolinon oder deren Salze zur Verwendung zur Behandlung von exzessiven oder anormalen Zellproliferationen.

Gegenstand der vorliegenden Erfindung ist auch die Verwendung der Verbindung 3-Z-[1-(4-(N-((4-Methyl-piperazin-1-yl)-methylcarbonyl)-N-methyl-amino)-anilino)-1-phenyl-methylene]-6-methoxycarbonyl-2-indolinon oder deren Salze zur Herstellung eines Arzneimittels, welches zur Behandlung von exzessiven oder anormalen Zellproliferationen geeignet ist.

Gegenstand der vorliegenden Erfindung ist auch ein Verfahren zur Herstellung eines Arzneimittels, dadurch gekennzeichnet, daß auf nichtchemischem Wege die Verbindung 3-z-[1-(4-(N-((4-Methyl-piperazin-1-yl)-methylcarbonyl)-N-methyl-amino)-anilino)-1-phenyl-methylene]-6-methoxycarbonyl-2-indolinon oder deren Salze in einen oder mehrere inerte Trägerstoffe und/oder Verdünnungsmittel eingearbeitet wird.

WO-A-99/15500 offenbart substituierte Indolinone, und insbesondere 3-(Anilinomethylen)-2-indolinone, und ihre Verwendung als Protein Tyrosin Kinase und Serin/Threonin Kinase Inhibitoren.

US-A-5,886,020 offenbart 3-Phenylmethylen-2-indolinone und ihre Verwendung für die Modulierung, Regulierung und/oder Inhibierung von Tyrosin Kinase Signale.

DE-A-19815020 offenbart 3-(Anilinomethylen)-2-indolinone und ihre Verwendung als Arzneimittels, welches zur Behandlung von exzessiven oder anormalen Zellproliferationen geeignet ist.

Die Erfindungsgemäße Verbindung gehört zu einer Gruppe von in 6-Stellung substituierte Indolinone der allgemeinen Formel 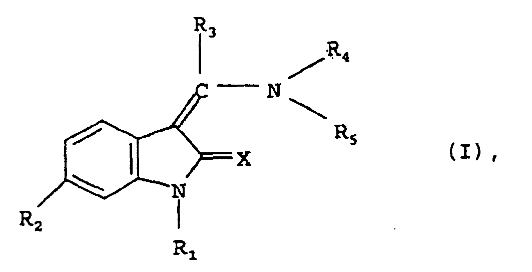 die als solches selbst nicht zur vorliegende Erfindung gehören, deren Tautomere, deren Diastereomere, deren Enantiomere, deren Gemische und deren Salze, insbesondere deren physiologisch verträgliche Salze, welche wertvolle Eigenschaften aufweisen.

Die obigen Verbindungen der allgemeinen Formel I, in der R₁ ein Wasserstoffatom oder einen Prodrugrest darstellt, weisen wertvolle pharmakologische Eigenschaften auf, insbesondere eine inhibierende Wirkung auf verschiedene Kinasen, vor allem auf Rezeptor-Tyrosinkinasen wie VEGFR2, PDGFRα, PDGFRβ, FGFR1, FGFR3, EGFR, HER2, IGF1R und HGFR, sowie auf Komplexe von CDK's (Cyclin Dependent Kinases) wie CDK1, CDK2, CDK3, CDK4, CDK5, CDK6, CDK7, CDK8 und CDK9 mit ihren spezifischen Cyclinen (A, B1, B2, C, D1, D2, D3, E, F, G1, G2, H, I und K) und auf virales Cyclin (siehe L. Mengtao in J. Virology 71(3), 1984-1991 (1997)), sowie auf die Proliferation kultivierter humaner Zellen, insbesondere die von Endothelzellen, z.B. bei der Angiogenese, aber auch auf die Proliferation anderer Zellen, insbesondere von Tumorzellen.

Die übrigen Verbindungen der obigen allgemeinen Formel I, in der R₁ kein Wasserstoffatom und keinen Prodrugrest darstellt, stellen wertvolle zwischenprodukte zur Herstellung der vorstehend erwähnten Verbindungen dar.

In der obigen allgemeinen Formel I bedeuten
X ein Sauerstoff- oder Schwefelatom,
R₁ ein Wasserstoffatom oder einen Prodrugrest wie eine C₁₋₄-Alkoxycarbonyl- oder C₂₋₄-Alkanoylgruppe,
R₂ eine Carboxygruppe, eine lineare oder verzweigte C₁₋₆-Alkoxy-carbonylgruppe, eine C₄₋₇-Cycloalkoxy-carbonyl- oder eine Aryloxycarbonylgruppe,
eine lineare oder verzweigte C₁₋₆-Alkoxy-carbonylgruppe, die im Alkylteil terminal durch eine Phenyl-, Heteroaryl-, Carboxy-, C₁₋₃-Alkoxy-carbonyl-, Aminocarbonyl-, C₁₋₃-Alkylamino-carbonyl- oder Di-(C₁₋₃-Alkyl)-aminocarbonylgruppe substituiert ist,
eine lineare oder verzweigte C₂₋₆-Alkoxy-carbonylgruppe, die im Alkylteil terminal durch ein Chloratom oder eine Hydroxy-, C₁₋₃-Alkoxy-, Amino-, C₁₋₃-Alkylamino- oder Di-(C₁₋₃-Alkyl)-aminogruppe substituiert ist,
eine Aminocarbonyl- oder Methylaminocarbonylgruppe, eine in 2-Position der Ethylgruppe gegebenfalls durch eine Hydroxy- oder C₁₋₃-Alkoxygruppe substituierte Ethylaminocarbonylgruppe oder,
sofern R₄ keine Aminosulfonyl-phenyl- oder N-(C₁₋₅-Alkyl) - C₁₋₃-alkylaminocarbonyl-phenylgruppe darstellt, auch eine Di-(C₁₋₂-Alkyl) -aminocarbonylgruppe,
R₃ ein Wasserstoffatom, eine C₁₋₆-Alkyl-, C₃₋₇-Cycloalkyl-, Trifluormethyl- oder Heteroarylgruppe,
eine Phenyl- oder Naphthylgruppe, eine durch ein Fluor-, Chlor-, Brom- oder Iodatom, durch eine Trifluormethyl-, C₁₋₃-Alkyl- oder C₁₋₃-Alkoxygruppe mono- oder disubstituierte Phenyl- oder Naphthylgruppe, wobei im Fall der Disubstitution die Substituenten gleich oder verschieden sein können und wobei die vorstehend genannten unsubstituierten sowie die mono- und disubstituierten Phenyl- und Naphthylgruppen zusätzlich
durch eine Hydroxy-, Hydroxy-C₁₋₃-alkyl- oder C₁₋₃-Alkoxy-C₁₋₃-alkylgruppe,
durch eine Cyano-, Carboxy-, Carboxy-C₁₋₃-alkyl-, C₁₋₃-Alkoxycarbonyl-, Aminocarbonyl-, C₁₋₃-Alkylamino-carbonyl- oder Di-(C₁₋₃-alkyl)-aminocarbonylgruppe,
durch eine Nitrogruppe,
durch eine Amino-, C₁₋₃-Alkylamino-, Di-(C₁₋₃-alkyl)-amino- oder Amino-C₁₋₃-alkylgruppe,
durch eine C₁₋₃-Alkylcarbonylamino-, N-(C₁₋₃-Alkyl)-C₁₋₃-alkylcarbonylamino-, C₁₋₃-Alkylcarbonylamino-C₁₋₃-alkyl-, N-(C₁₋₃-Alkyl) -C₁₋₃-alkylcarbonylamino-C₁₋₃-alkyl-, C₁₋₃- Alkylsulfonylamino-, C₁₋₃)-Alkylsulfonylamino-C₁₋₃-alkyl-, N- (C₁₋₃-Alkyl) -C₁₋₃-alkylsulfonylamino-C₁₋₃]-alkyl- oder Aryl-C₁₋₃-alkylsulfonylaminogruppe,
durch eine Cycloalkylamino-, Cycloalkylenimino-, Cycloalkyleniminocarbonyl-, Cycloalkylenimino-C₁₋₃-alkyl-, Cycloalkyleniminocarbonyl-C₁₋₃-alkyl- oder Cycloalkylen-iminosulfonyl-C₁₋₃-alkylgruppe mit jeweils 4 bis 7 Ringgliedern, wobei jeweils die Methylengruppe in Position 4 in einer 6- oder 7-gliedrigen Cycloalkyleniminogruppe durch ein Sauerstoff- oder Schwefelatom, durch eine Sulfinyl-, Sulfonyl-, -NH- oder -N(C₁₋₃-Alkyl)-Gruppe ersetzt sein kann,
oder durch eine Heteroaryl- oder Heteroaryl-C₁₋₃-alkylgruppe substituiert sein kann,
R₄ eine C₃₋₇-Cycloalkylgruppe,
wobei die Methylengruppe in Position 4 einer 6- oder 7-gliedrigen Cycloalkylgruppe durch eine Amino-, C₁₋₃-Alkylamino- oder Di-(C₁₋₃-alkyl) -aminogruppe substituiert oder durch eine -NH- oder -N(C₁₋₃-Alkyl)-Gruppe ersetzt sein kann,
oder eine durch die Gruppe R₆ substituierte Phenylgruppe, die zusätzlich durch Fluor-, Chlor-, Brom- oder Iodatome, durch C₁₋₅-Alkyl-, Trifluormethyl-, Hydroxy-, C₁₋₃-Alkoxy-, Carboxy-, C₁₋₃-Alkoxycarbonyl-, Amino-, Acetylamino-, C₁₋₃-Alkyl-sulfonyl-amino-, Aminocarbonyl-, C₁₋₃-Alkyl-aminocarbonyl-, Di-(C₁₋₃-alkyl)-aminocarbonyl-, Aminosulfonyl-, C₁₋₃-Alkyl-aminosulfonyl-, Di-(C₁₋₃-alkyl)-aminosulfonyl-, Nitro- oder Cyanogruppen mono- oder disubstituiert sein kann, wobei die Substituenten gleich oder verschieden sein können und wobei
R₆ ein Wasserstoff-, Fluor-, Chlor-, Brom- oder Iodatom,
eine Cyano-, Nitro-, Amino-, C₁₋₅-Alkyl-, C₃₋₇-Cycloalkyl-, Trifluormethyl-, Phenyl-, Tetrazolyl- oder Heteroarylgruppe,
die Gruppe der Formel 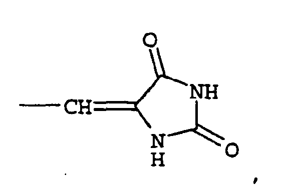 in der die an ein Stickstoffatom gebundenen Wasserstoffatome unabhängig voneinander jeweils durch eine C₁₋₃-Alkylgruppe ersetzt sein können,
eine C₁₋₃-Alkoxygruppe, eine C₁₋₃-Alkoxy-C₁₋₃-alkoxy-, Phenyl-C₁₋₃-alkoxy-, Amino-C₂₋₂-alkoxy-, C₁₋₃-Alkylamino-C₂₋₃-alkoxy-, Di- (C₁₋₃-alkyl) -amino-C₂₋₃-alkoxy-, Phenyl-C₁₋₃-alkylamino-C₂₋₃-alkoxy-, N- (C₁₋₃-Alkyl) -phenyl-C₁₋₃-alkylamino-C₂₋₃-alkoxy-, C₅₋₇-Cycloalkylenimino-C₂₋₃-alkoxy- oder C₁₋₃-Alkylmercaptogruppe,
eine Carboxy-, C₁₋₄-Alkoxycarbonyl-, Aminocarbonyl-, C₁₋₃-Alkylamino-carbonyl-, N- (C₁₋₅-Alkyl) -C₁₋₃-alkylaminocarbonyl-, Phenyl-C₁₋₃-alkylamino-carbonyl-, N- (C₁₋₃-Alkyl) -phenyl-C₁₋₃-alkylamino-carbonyl-, Piperazinocarbonyl- oder N-(C₁₋₃-Alkyl)-piperazinocarbonylgruppe,
eine C₁₋₃-Alkylaminocarbonyl- oder N- (C₁₋₅-Alkyl) -C₁₋₃-alkylaminocarbonylgruppe, in denen ein Alkylteil durch eine Carboxy- oder C₁₋₃-Alkoxycarbonylgruppe oder in 2- oder 3-Stellung durch eine Di-(C₁₋₃-alkyl)-amino-, Piperazino-, N-(C₁₋₃-Alkyl)-piperazino- oder eine 4- bis 7-gliedrige Cycloalkyleniminogruppe substituiert ist,
eine C₃₋₇-Cycloalkyl-carbonylgruppe,
wobei die Methylengruppe in Position 4 des 6- oder 7-gliedrigen Cycloalkylteils durch eine Amino-, C₁₋₃-Alkylamino- oder Di-(C₁₋₃-alkyl)-aminogruppe substituiert oder durch eine -NH- oder -N(C₁₋₃-Alkyl)-Gruppe ersetzt sein kann,
eine 4- bis 7-gliedrige Cycloalkyleniminogruppe, in der eine mit der Iminogruppe verknüpfte Methylengruppe durch eine Carbonyl- oder Sulfonylgruppe ersetzt sein kann oder
der Cycloalkylenteil mit einem Phenylring kondensiert sein kann oder
ein oder zwei Wasserstoffatome jeweils durch eine C₁₋₃-Alkylgruppe ersetzt sein können oder/und
jeweils die Methylengruppe in Position 4 einer 6- oder 7-gliedrigen Cycloalkyleniminogruppe durch eine Carboxy-, C₁₋₃-Alkoxycarbonyl-, Aminocarbonyl-, C₁₋₃-Alkylaminocarbonyl-, Di-(C₁₋₃-alkyl) -aminocarbonyl-, Phenyl-C₁₋₃-alkylamino- oder N- (C₁₋₃-Alkyl) -phenyl-C₁₋₃-alkylaminogruppe substituiert oder
durch ein Sauerstoff- oder Schwefelatom, durch eine Sulfinyl-, Sulfonyl-, -NH-, -N(C₁₋₃-Alkyl)-, -N(Phenyl)-, -N(C₁₋₃-Alkyl-carbonyl)- oder -N(Benzoyl)- Gruppe ersetzt sein kann,
eine durch die Gruppe R₇ substituierte C₁₋₄-Alkylgruppe, wobei
R₇ eine C₃₋₇-Cycloalkylgruppe,
wobei die Methylengruppe in Position 4 einer 6- oder 7-gliedrigen Cycloalkylgruppe durch eine Amino-, C₁₋₃-Alkylamino- oder Di-(C₁₋₃-alkyl)-aminogruppe substituiert oder durch eine -NH- oder -N(C₁₋₃-Alkyl) -Gruppe ersetzt sein kann oder
in einer 5- bis 7-gliedrigen Cycloalkylgruppe eine -(CH₂)₂-Gruppe durch eine -CO-NH-Gruppe ersetzt sein kann, eine -(CH₂)₃-Gruppe durch eine -NH-CO-NH- oder -CO-NH-CO-Gruppe ersetzt sein kann oder eine -(CH₂)₄-Gruppe durch eine -NH-CO-NH-CO-Gruppe ersetzt
sein kann, wobei jeweils ein an ein Stickstoffatom gebundenes Wasserstoffatom durch eine C₁₋₃-Alkylgruppe ersetzt sein kann,
eine Aryl- oder Heteroarylgruppe,
eine Hydroxy- oder C₁₋₃-Alkoxygruppe,
eine Amino-, C₁₋₇-Alkylamino-, Di-(C₁₋₇-Alkyl) -amino-, Phenylamino-, N-Phenyl-C₁₋₃-alkyl-amino-, Phenyl-C₁₋₃-alkylamino-, N-(C₁₋₃-Alkyl)-phenyl-C₁₋₃-alkylamino- oder Di- (phenyl-C₁₋₃-alkyl)-aminogruppe,
eine ω-Hydroxy-C₂₋₃-alkyl-amino-, N-(C₁₋₃-Alkyl)-ω-hydroxy-C₂₋₃-alkyl-amino-, Di-(ω-Hydroxy-C₂₋₃-alkyl) -amino-, Di-(ω)-(C₁₋₃-Alkoxy)-C₂₋₃-alkyl) -amino- oder N- (Dioxolan-2-yl)-C₁₋₃-alkyl-aminogruppe,
eine C₁₋₃-Alkylcarbonylamino-C₂₋₃-alkyl-amino- oder C₁₋₃-Alkylcarbonylamino-C₂₋₃-alkyl-N- (C₁₋₃-alkyl) -aminogruppe,
eine C₁₋₃-Alkylsulfonylamino-, N-(C₁₋₃-Alkyl)-C₁₋₃-alkylsulfonylamino-, C₁₋₃-Alkylsulfonylamino-C₂₋₃-alkyl-amino- oder C₁₋₃-Alkylsulfonylamino-C₂₋₃-alkyl-N- (C₁₋₃-alkyl) -aminogruppe,
eine Hydroxycarbonyl-C₁₋₃-alkylamino- oder N-(C₁₋₁-Alkyl)-hydroxycarbonyl-C₁₋₃-alkyl-aminogruppe,
eine Guanidinogruppe, in der ein oder zwei Wasserstoffatome jeweils durch eine C₁₋₃-Alkylgruppe ersetzt sein können,
eine Gruppe der Formel

-N(R₈)-CO-(CH₂)ₙ-R₉ (II),

in der
R₈ ein Wasserstoffatom oder eine C₁₋₃-Alkylgruppe,
n eine der Zahlen 0, 1, 2 oder 3 und
R₉ eine Amino-, C₁₋₄-Alkylamino-, Di- (C₁₋₄-Alkyl) -amino-, Phenylamino-, N-(C₁₋₄-Alkyl)-phenylamino-, Benzylamino-, N-(C₁₋₄-Alkyl)-benzylamino- oder C₁₋₄-Alkoxygruppe, eine 4- bis 7-gliedrige Cycloalkyleniminogruppe, wobei jeweils die Methylengruppe in Position 4 einer 6- oder 7-gliedrigen Cycloalkyleniminogruppe durch ein Sauerstoff- oder Schwefelatom, durch eine Sulfinyl-, Sulfonyl-, -NH-, -N(C₁₋₃-Alkyl)-, -N(Phenyl)-, -N(C₁₋₃-Alkyl-carbonyl)- oder -N(Benzoyl)- Gruppe ersetzt sein kann, oder, sofern n eine der Zahlen 1, 2 oder 3 darstellt, auch ein Wasserstoffatom bedeuten,
eine Gruppe der Formel

-N(R₁₀)-(CH₂)ₘ-(CO)ₒ-R₁₁ (III),

in der
R₁₀ ein Wasserstoffatom, eine C₁₋₃-Alkylgruppe, eine C₁₋₃-Alkylcarbonyl-, Arylcarbonyl-, Phenyl-C₁₋₃-alkylcarbonyl-, C₁₋₃-Alkylsulfonyl-, Arylsulfonyl- oder Phenyl-C₁₋₃-alkylsulfonylgruppe,
m eine der Zahlen 1, 2, 3 oder 4,
o die Zahl 1 oder, sofern m eine der Zahlen 2, 3 oder 4 ist, auch die Zahl 0 und
R₁₁ eine Amino-, C₁₋₄-Alkylamino-, Di-(C₁₋₄-Alkyl)-amino-, Phenylamino-, N-(C₁₋₄-Alkyl)-phenylamino-, Benzylamino-, N- (C₁₋₄-Alkyl) -benzylamino-, C₁₋₄-Alkoxy- oder C₁₋₃-Alkoxy-C₁₋₃)-alkoxygruppe, eine in 1-Position gegebenenfalls durch eine C₁₋₃-Alkylgruppe substituierte Di-(C₁₋₄-Alkyl)-amino-C₁₋₃-alkylaminogruppe oder eine 4- bis 7-gliedrige Cycloalkyleniminogruppe, wobei der Cycloalkylenteil mit einem Phenylring kondensiert sein kann oder jeweils die Methylengruppe in Position 4 einer 6- oder 7-gliedrigen Cycloalkyleniminogruppe durch ein Sauerstoff- oder Schwefelatom, durch eine Sulfinyl-, Sulfonyl-, -NH-, -N(C₁₋₃-Alkyl) -, -N(Phenyl)-, -N(C₁₋₃-Alkyl-carbonyl) - oder -N(Benzoyl)- Gruppe ersetzt sein kann, bedeuten,
eine C₄₋₇-Cycloalkylamino-, C₄₋₇-Cycloalkyl-C₁₋₃-alkylamino- oder C₄₋₇-Cycloalkenylaminogruppe, in der die Position 1 des Rings nicht an der Doppelbindung beteiligt ist und wobei die vorstehend genannten Gruppen jeweils zusätzlich am Aminstickstoffatom durch eine C₅₋₇-Cycloalkyl-, C₂₋₄ -Alkenyl- oder C₁₋₄-Alkylgruppe substituiert sein können,
eine 4- bis 7-gliedrige Cycloalkyleniminogruppe, in der
der Cycloalkylenteil mit einer Phenylgruppe oder mit einer gegebenenfalls durch ein Fluor-, Chlor-, Brom- oder Iodatom, durch eine Nitro-, C₁₋₃-Alkyl-, C₁₋₃-Alkoxy- oder Aminogruppe substituierten Oxazolo-, Imidazolo-, Thiazolo-, Pyridino-, Pyrazino- oder Pyrimidinogruppe kondensiert sein kann oder/und
ein oder zwei Wasserstoffatome jeweils durch eine C₁₋₃-Alkyl-, C₅₋₇-Cycloalkyl- oder Phenylgruppe ersetzt sein können oder/und
die Methylengruppe in Position 3 einer 5-gliedrigen Cycloalkyleniminogruppe durch eine Hydroxy-, Hydroxy-C₁₋₃-alkyl-, C₁₋₃-Alkoxy- oder C₁₋₃-Alkoxy-C₁₋₃-alkylgruppe substituiert sein kann,
jeweils die Methylengruppe in Position 3 oder 4 einer 6- oder 7-gliedrigen Cycloalkyleniminogruppe durch eine Hydroxy-, Hydroxy-C₁₋₃-alkyl-, C₁₋₃-Alkoxy-, C₁₋₃-Alkoxy-C₁₋₃-alkyl-, Carboxy-, C₁₋₄-Alkoxycarbonyl-, Amino-carbonyl-, C₁₋₃-Alkylaminocarbonyl-, Di-(C₁₋₃-alkyl) - aminocarbonyl-, Phenyl-C₁₋₃-alkylamino- oder N-(C₁₋₃-Alkyl)-phenyl-C₁₋₃-alkylaminogruppe substituiert oder
durch ein Sauerstoff- oder Schwefelatom, durch eine Sulfinyl-, Sulfonyl-, -NH-, -N(C₁₋₃-Alkyl-)-, -N(Phenyl)-, -N(Phenyl-C₁₋₃-alkyl-)-, -N(C₁₋₃-Alkyl-carbonyl-)-, -N(C₁₋₄-Hydroxy-carbonyl-)-, -N(C₁₋₄-Alkoxy-carbonyl-)-, -N(Benzoyl-)- oder -N(Phenyl-C₁₋₃-alkyl-carbonyl-)-Gruppe ersetzt sein kann,
wobei eine mit einem Imino-Stickstoffatom der Cycloalkyleniminogruppe verknüpfte Methylengruppe durch eine Carbonyl- oder Sulfonylgruppe ersetzt sein kann oder in einer 5- bis 7-gliedrigen monocyclischen oder mit einer Phenylgruppe kondensierten Cycloalkyleniminogruppe beide mit dem Imino-Stickstoffatom verknüpften Methylengruppen jeweils durch eine Carbonylgruppe ersetzt sein können,
bedeutet,
oder R₆ eine C₁₋₄-Alkylgruppe, die durch eine Carboxy-, C₁₋₃-Alkoxycarbonyl-, Aminocarbonyl-, C₁₋₃-Alkylaminocarbonyl- oder Di-(C₁₋₃-alkyl)-aminocarbonylgruppe oderdurch eine 4- bis 7-gliedrige Cycloalkyleniminocarbonyl-gruppe substituiert ist,
eine N- (C₁₋₃-Alkyl) -C₁₋₄-alkanoylaminogruppe, die im Alkylteil zusätzlich durch eine Carboxy- oder C₁₋₃-Alkoxycarbonylgruppe substituiert ist,
eine Gruppe der Formel

-N(R₁₂)-CO-(CH₂)ₚ-R₁₃ (IV),

in der
R₁₂ ein Wasserstoffatom, eine C₁₋₆-Alkyl- oder C₃₋₇-Cycloalkylgruppe oder eine terminal durch eine Phenyl-, Heteroaryl-, Trifluormethyl-, Hydroxy-, C₁₋₃-Alkoxy-, Aminocarbonyl-, C₁₋₄-Alkylamino-carbonyl-, Di-(C₁₋₄-alkyl) - amino-carbonyl-, C₁₋₃-Alkyl-carbonyl-, C₁₋₃-Alkyl-sulfonyl-amino-, N- (C₁₋₃-Alkyl) -C₁₋₃-alkyl-sulfonylamino-, C₁₋₃-Alkyl-aminosulfonyl- oder Di-(C₁₋₃-Alkyl)-aminosulfonyl-gruppe substituierte C₁₋₃-Alkylgruppe und
p eine der Zahlen 0, 1, 2 oder 3 darstellen und
R₁₃ die Bedeutungen der vorstehend erwähnten Gruppe R, annimmt, oder, sofern p eine der Zahlen 1, 2 oder 3 darstellt, auch ein Wasserstoffatom bedeutet,
eine Gruppe der Formel

-N(R₁₄)-(CH₂)_{q}-(CO)ᵣ-R₁₅ (V),

in der
R₁₄ ein Wasserstoffatom, eine C₁₋₄-Alkylgruppe, eine C₁₋₃-Alkylcarbonyl-, Arylcarbonyl-, Phenyl-C₁₋₃-alkylcarbonyl-, Heteroarylcarbonyl-, Heteroaryl-C₁₋₃-alkylcarbonyl-, C₁₋₄-Alkylsulfonyl-, Arylsulfonyl-, Phenyl-C₁₋₃-alkylsulfonyl-, Heteroarylsulfonyl- oder Heteroaryl-C₁₋₃-alkyl-sulfonylgruppe,
q eine der Zahlen 1, 2, 3 oder 4,
r die Zahl 1 oder, sofern q eine der Zahlen 2, 3 oder 4 ist, auch die Zahl 0 darstellen und
R₁₅ die Bedeutungen der vorstehend erwähnten Gruppe R₇ annimmt,
eine Gruppe der Formel

-N(R₁₆)-SO₂-R₁₇ (VI),

in der
R₁₆ ein Wasserstoffatom oder eine terminal gegebenenfalls durch eine Cyano-, Trifluormethyl-carbonyl-amino- oder N-(C₁₋₃-Alkyl)-trifluormethyl-carbonyl-aminogruppe substituierte C₁₋₄-Alkylgruppe und
R₁₇ eine C₁₋₃-Alkylgruppe bedeuten,
eine durch eine Di-(C₁₋₃-Alkyl)-amino-C₁₋₃-alkyl-carbonyl- oder Di-(C₁₋₃-Alkyl)-amino-C₁₋₃-alkyl-sulfonylgruppe und eine Di- (C₁₋₃-Alkyl) -aminocarbonyl-C₁₋₃-alkylgruppe substituierte Aminogruppe,
oder eine N-(C₁₋₃-Alkyl)-C₁₋₅-alkylsulfonylamino- oder N-(C₁₋₃-Alkyl)-phenylsulfonylaminogruppe, in denen der Alkylteil zusätzlich durch eine Cyano- oder Carboxygruppe substituiert ist,
wobei alle in den unter R₆ genannten Resten enthaltenen einfach gebundenen oder ankondensierten Phenylgruppen durch Fluor-, Chlor-, Brom- oder Iodatome, durch C₁₋₅-Alkyl-, Trifluormethyl-, Hydroxy-, C₁₋₃-Alkoxy-, Carboxy-, C₁₋₃-Alkoxycarbonyl-, Aminocarbonyl-, C₁₋₄-Alkylamino-carbonyl-, Di-(C₁₋₄-alkyl)-amino-carbonyl-, Aminosulfonyl-, C₁₋₃-Alkyl-aminosulfonyl-, Di-(C₁₋₃-Alkyl)-aminosulfonyl-, C₁₋₃-Alkyl-sulfonylamino-, Nitro- oder Cyanogruppen mono- oder disubstituiert sein können, wobei die Substituenten gleich oder verschieden sein können, oder zwei benachbarte Wasserstoffatome der Phenylgruppen durch eine Methylendioxygruppe ersetzt sein können,
und
R₅ ein Wasserstoffatom oder eine C₁₋₃-Alkylgruppe,
wobei unter dem Ausdruck eine Arylgruppe eine gegebenenfalls durch ein Fluor-, Chlor-, Brom- oder Iodatom, durch eine Cyano-, Trifluormethyl-, Nitro-, Carboxy-, Aminocarbonyl-, C₁₋₃-Alkyl- oder C₁₋₃-Alkoxygruppe mono- oder disubstituierte Phenyl- oder Naphthylgruppe und
unter dem Ausdruck eine Heteroarylgruppe eine im Kohlenstoffgerüst gegebenenfalls durch eine C₁₋₃-Alkylgruppe substituierte monocyclische 5- oder 6-gliedrige Heteroarylgruppe, wobei
die 6-gliedrige Heteroarylgruppe ein, zwei oder drei Stickstoffatome und
die 5-gliedrige Heteroarylgruppe eine gegebenenfalls durch eine C₁₋₃-Alkyl- oder Phenyl-C₁₋₃-alkylgruppe substituierte Iminogruppe, ein Sauerstoff- oder Schwefelatom oder
eine gegebenenfalls durch eine C₁₋₃-Alkyl- oder Phenyl-C₁₋₃-alkylgruppe substituierte Iminogruppe oder ein Sauerstoff- oder Schwefelatom und zusätzlich ein Stickstoffatom oder
eine gegebenenfalls durch eine C₁₋₃-Alkyl- oder Phenyl-C₁₋₃-alkylgruppe substituierte Iminogruppe und zwei Stickstoffatome enthält,
und außerdem an die vorstehend erwähnten monocyclischen heterocyclischen Gruppen über zwei benachbarte Kohlenstoffatome ein Phenylring ankondensiert sein kann und die Bindung über ein Stickstoffatom oder über ein Kohlenstoffatom des heterocyclischen Teils oder eines ankondensierten Phenylrings erfolgt,
zu verstehen ist,
die Wasserstoffatome in den vorstehend genannten Alkyl- und Alkoxygruppen oder in den in vorstehend definierten Gruppen der Formel I enthaltenen Alkylteilen teilweise oder ganz durch Fluoratome ersetzt sein können,
die in den vorstehend definierten Gruppen vorhandenen gesättigten Alkyl- und Alkoxyteile, die mehr als 2 Kohlenstoffatome enthalten, auch deren verzweigte Isomere, wie beispielsweise die Isopropyl-, tert.Butyl-, Isobutylgruppe, einschließen, sofern nichts anderes erwähnt wurde, und
wobei zusätzlich das Wasserstoffatom einer vorhandenen Carboxygruppe oder ein an ein Stickstoffatom gebundenes Wasserstoffatom, beispielsweise einer Amino-, Alkylamino- oder Iminogruppe oder eines gesättigten N-Heterocyclus wie der Piperidinylgruppe, jeweils durch einen in-vivo abspaltbaren Rest ersetzt sein kann.

Unter einem von einer Imino- oder Aminogruppe in-vivo abspaltbaren Rest ist beispielsweise eine Hydroxygruppe, eine Acylgruppe wie die Benzoyl- oder Pyridinoylgruppe oder eine C₁₋₁₆-Alkanoylgruppe wie die Formyl-, Acetyl-, Propionyl-, Butanoyl-, Pentanoyl- oder Hexanoylgruppe, eine Allyloxycarbonylgruppe, eine C₁₋₁₆-Alkoxycarbonylgruppe wie die Methoxycarbonyl-, Ethoxycarbonyl-, Propoxycarbonyl-, Isopropoxycarbonyl-, Butoxycarbonyl-, tert.Butoxycarbonyl-, Pentoxycarbonyl-, Hexyloxycarbonyl-, Octyloxycarbonyl-, Nonyloxycarbonyl-, Decyloxycarbonyl-, Undecyloxycarbonyl-, Dodecyloxycarbonyl- oder Hexadecyloxycarbonylgruppe, eine Phenyl-C₁₋₆-alkoxycarbonylgruppe wie die Benzyloxycarbonyl-, Phenylethoxycarbonyl- oder Phenylpropoxycarbonylgruppe, eine C₁₋₃-Alkylsulfonyl-C₂₋₄-alkoxycarbonyl-, C₁₋₃-Alkoxy-C₂₋₄-alkoxy-C₂₋₄-alkoxycarbonyl- oder RₑCO-O-(R_{f}CR_{g})-O-CO-Gruppe, in der
Rₑ eine C₁₋₈-Alkyl-, C₅₋₇-Cycloalkyl-, Phenyl- oder Phenyl-C₁₋₃-alkylgruppe,
R_{f} ein Wasserstoffatom, eine C₁₋₃-Alkyl-, C₅₋₇-Cycloalkyl- oder Phenylgruppe und
R_{g} ein Wasserstoffatom, eine C₁₋₃-Alkyl- oder RₑCO-O- (R_{f}CR_{g}) - O-Gruppe, in der Rₑ bis R_{g} wie vorstehend erwähnt definiert sind, darstellen,
wobei zusätzlich für eine Aminogruppe die Phthalimidogruppe in Betracht kommt, zu verstehen, wobei die vorstehend erwähnten Esterreste ebenfalls als in-vivo in eine Carboxygruppe überführbare Gruppe verwendet werden können.

Nach folgenden im Prinzip literaturbekannten Verfahren können die Verbindungen der Formel (I), bezeichungsweise kann die Erfindungsgemäße Verbindung, hergestellt werden:
a. Umsetzung einer Verbindung der allgemeinen Formel 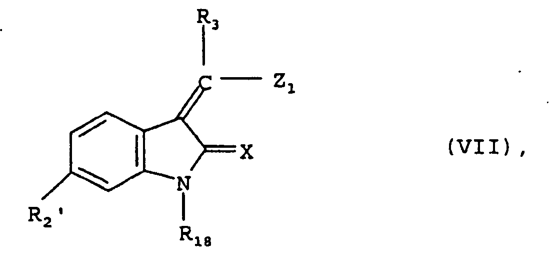 in der
   X und R₃ wie eingangs erwähnt definiert sind,
   R₂' die für R₂ eingangs erwähnten Bedeutungen besitzt,
   R₁₈ ein Wasserstoffatom oder eine Schutzgruppe für das Stickstoffatom der Lactamgruppe, wobei einer der Reste R₂' und R₁₈ auch eine gegebenenenfalls über einen Spacer gebildete Bindung an eine Festphase darstellen kann und der andere der Reste R₂' und R₁₈ die vorstehend erwähnten Bedeutungen besitzt, und Z₁ ein Halogenatom, eine Hydroxy-, Alkoxy- oder Aryl-alkoxygruppe, z.B. ein Chlor- oder Bromatom, eine Methoxy-, Ethoxy- oder Benzyloxygruppe, bedeuten,
   mit einem Amin der allgemeinen Formel 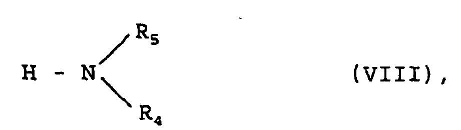 in der
   R₄ und R₅ wie eingangs erwähnt definiert sind,
   und erforderlichenfalls anschließende Abspaltung einer verwendeten Schutzgruppe für das Stickstoffatom der Lactamgruppe oder von einer Festphase.
   Als Schutzgruppe für das Stickstoffatom der Lactamgruppe kommt beispielsweise eine Acetyl-, Benzoyl-, Ethoxycarbonyl-, tert.-Butyloxycarbonyl- oder Benzyloxycarbonylgruppe und
   als Festphase ein Harz wie ein 4-(2',4'-Dimethoxyphenylaminomethyl)-phenoxyharz, wobei die Bindung zweckmäßigerweise über die Aminogruppe erfolgt, oder ein p-Benzyloxybenzylalkoholharz, wobei die Bindung zweckmäßigerweise über ein Zwischenglied wie ein 2,5-Dimethoxy-4-hydroxy-benzylderivat erfolgt, in Betracht.
   Die Umsetzung wird zweckmäßigerweise in einem Lösungsmittel wie Dimethylformamid, Toluol, Acetonitril, Tetrahydrofuran, Dimethylsulfoxid, Methylenchlorid oder deren Gemischen gegebenenfalls in Gegenwart einer inerten Base wie Triethylamin, N-Ethyl-diisopropylamin oder Natriumhydrogencarbonat bei Temperaturen zwischen 20 und 175°C durchgeführt, wobei eine verwendete Schutzgruppe infolge Umamidierung gleichzeitig abgespalten werden kann.
   Bedeutet Z₁ in einer Verbindung der allgemeinen Formel VII ein Halogenatom, dann wird die Umsetzung vorzugsweise in Gegenwart einer inerten Base bei Temperaturen zwischen 20 und 120°C, durchgeführt.
   Bedeutet Z₁ in einer Verbindung der allgemeinen Formel VII eine Hydroxy-, Alkoxy- oder Arylalkoxygruppe, dann wird die Umsetzung vorzugsweise bei Temperaturen zwischen 20 und 200°C, durchgeführt.
   Die gegebenenfalls erforderliche anschließende Abspaltung einer verwendeten Schutzgruppe wird zweckmäßigerweise entweder hydrolytisch in einem wäßrigen oder alkoholischen Lösungsmittel, z.B. in Methanol/Wasser, Ethanol/Wasser, Isopropanol/Wasser, Tetrahydrofuran/Wasser, Dioxan/Wasser, Dimethylformamid/- Wasser, Methanol oder Ethanol in Gegenwart einer Alkalibase wie Lithiumhydroxid, Natriumhydroxid oder Kaliumhydroxid bei Temperaturen zwischen 0 und 100°C, vorzugsweise bei Temperaturen zwischen 10 und 50°C,
   oder vorteilhafterweise durch Umamidierung mit einer organischen Base wie Ammoniak, Butylamin, Dimethylamin oder Piperidin in einem Lösungsmittel wie Methanol, Ethanol, Dimethylformamid und deren Gemischen oder in einem Überschuß des eingesetzten Amins bei Temperaturen zwischen 0 und 100°C, vorzugsweise bei Temperaturen zwischen 10 und 50°C, durchgeführt.
   Die Abspaltung von einer verwendeten Festphase erfolgt vorzugsweise mittels Trifluoressigsäure und Wasser bei Temperaturen zwischen 0 und 35°C, vorzugsweise bei Raumtemperatur.
b. Zur Herstellung einer Verbindung der allgemeinen Formel I, in der R₂ mit Ausnahme der Carboxygruppe wie eingangs erwähnt definiert ist: Umsetzung einer Verbindung der allgemeinen Formel 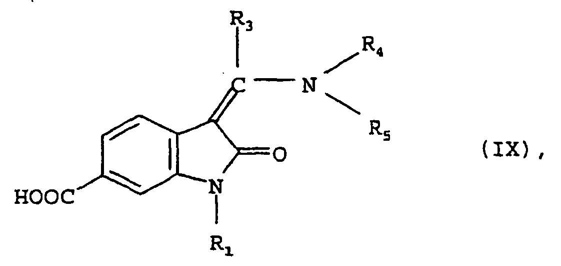 in der
   R₁ und R₃ bis R₅ wie eingangs erwähnt definiert sind, oder deren reaktionsfähigen Derivaten mit einer Verbindung der allgemeinen Formel

   H - R₁₉ (X),

   in der
   R₁₉ ein C₁₋₆-Alkanol, ein C₄₋₇-Cycloalkanol oder ein aromatischer Alkohol,
   ein C₁₋₆-Alkanol, der im Alkylteil terminal durch eine Phenyl-, Heteroaryl-, Carboxy-, C₁₋₃-Alkoxy-carbonyl-, Aminocarbonyl-, C₁₋₃-Alkylamino-carbonyl- oder Di- (C₁₋₃-Alkyl) -aminocarbonylgruppe substituiert ist,
   ein C₂₋₆-Alkanol, der im Alkylteil terminal durch ein Chloratom oder eine Hydroxy-, C₂₋₃-Alkoxy-, Amino-, C₁₋₃-Alkylamino- oder Di-(C₁₋₃-Alkyl)-aminogruppe substituiert ist,
   eine Amino- oder Methylaminogruppe, eine in 2-Position der Ethylgruppe gegebenfalls durch eine Hydroxy- oder C₁₋₃-Alkoxygruppe substituierte Ethylaminogruppe oder eine Di-(C₁₋₂-Alkyl)-aminogruppe bedeutet.
   Die Veresterung oder Amidierung wird vorzugsweise in einem Lösungsmittel wie Methylenchlorid, Diethylether, Tetrahydrofuran, Toluol, Dioxan, Acetonitril, Dimethylsulfoxid oder Dimethylformamid gegebenenfalls in Gegenwart einer anorganischen oder einer tertiären organischen Base, vorzugsweise bei Temperaturen zwischen 20°C und der Siedetemperatur des verwendeten Lösungsmittel, durchgeführt. Hierbei wird die Umsetzung mit einer entsprechenden Säure vorzugsweise in Gegenwart eines wasserentziehenden Mittels, z.B. in Gegenwart von Chlorameisensäureisobutylester, Orthokohlensäuretetraethylester, Orthoessigsäuretrimethylester, 2,2-Dimethoxypropan, Tetramethoxysilan, Thionylchlorid, Trimethylchlorsilan, Phosphortrichlorid, Phosphorpentoxid, N,N'-Dicyclohexylcarbodiimid, N,N'-Dicyclohexylcarbodiimid/N-Hydroxysuccinimid, N,N'-Dicyclohexylcarbodiimid/1-Hydroxy-benztriazol, 2-(1H-Benzotriazol-1-yl)-1,1,3,3-tetramethyluronium-tetrafluorborat, 2-(1H-Benzotriazol-1-yl)-1,1,3,3-tetramethyluronium-tetrafluorborat/1-Hydroxy-benztriazol, N,N'-carbonyldiimidazol oder Triphenylphosphin/Tetrachlorkohlenstoff, und gegebenenfalls unter Zusatz einer Base wie Pyridin, 4-Dimethylaminopyridin, N-Methyl-morpholin oder Triethylamin zweckmäßigerweise bei Temperaturen zwischen 0 und 150°C, vorzugsweise bei Temperaturen zwischen 0 und 100°C, und die Acylierung mit einer entsprechenden reaktionsfähigen Verbindung wie deren Anhydrid, Ester, Imidazolide oder Halogenide gegebenenfalls in Gegenwart einer tertiären organischen Base wie Triethylamin, N-Ethyl-diisopropylamin oder N-Methyl-morpholin bei Temperaturen zwischen 0 und 150°C, vorzugsweise bei Temperaturen zwischen 50 und 100°C, durchgeführt.
c. Zur Herstellung einer Verbindung der allgemeinen Formel I, in der R₄ eine durch die Gruppe R₇ substituierte C₁₋₄-Alkylgruppe darstellt, wobei
   R₇ eine Amino-, C₁₋₇-Alkylamino-, Di-(C₁₋₇-Alkyl)-amino-, Phenylamino-, N-Phenyl-C₁₋₃-alkyl-amino-, Phenyl-C₁₋₃-alkylamino-, N-(C₁₋₃-Alkyl) -phenyl-C₁₋₃-alkylamino- oder Di- (phenyl-C₁₋₃-alkyl) -aminogruppe,
   eine ω-Hydroxy-C₂₋₃-alkyl-amino-, N-(C₁₋₃-Alkyl)-ω-hydroxy-C₂₋₃-alkyl-amino-, Di- (ω-Hydroxy-C₂₋₃-alkyl) -amino-, Di- (ω- (C₁₋₃-Alkoxy) -C₂₋₃-alkyl) -amino- oder N- (Dioxolan-2-yl) -C₁₋₃-alkyl-aminogruppe,
   eine C₁₋₃-Alkylcarbonylamino-C₂₋₃-alkyl-amino- oder C₁₋₃-Alkylcarbonylamino-C₂₋₃-alkyl-N-(C₂₋₃-alkyl) -aminogruppe,
   eine C₁₋₃-Alkylsulfonylamino-, N- (C₁₋₃-Alkyl) -C₁₋₃-alkylsulfonylamino-, C₁₋₃-Alkylsulfonylamino-C₂₋₃-alkyl-amino- oder
   C₁₋₃-Alkylsulfonylamino-C₂₋₃-alkyl-N- (C₁₋₃-alkyl) -aminogruppe,
   eine Gruppe der Formel

   -N(R₁₀)-(CH₂)ₘ-(CO)ₒ-R₁₁ (III),

   in der
   R₁₀ ein Wasserstoffatom, eine C₁₋₃-Alkylgruppe, eine C₁₋₃-Alkylcarbonyl-, Arylcarbonyl-, Phenyl-C₁₋₃-alkylcarbonyl-, C₁₋₃-Alkylsulfonyl-, Arylsulfonyl- oder Phenyl-C₁₋₃-alkylsulfonylgruppe,
   m eine der Zahlen 1, 2, 3 oder 4,
   o die Zahl 1 und
   R₁₁ eine Amino-, C₁₋₄-Alkylamino-, Di-(C₁₋₄-Alkyl)-amino-, Phenylamino-, N-(C₁₋₄-Alkyl)-phenylamino-, Benzylamino-, N- (C₁₋₄-Alkyl)-benzylamino-, C₁₋₄-Alkoxy- oder C₁₋₃-Alkoxy-C₁₋₃-alkoxygruppe, eine in 1-Position gegebenenfalls durch eine C₁₋₃-Alkylgruppe substituierte Di-(C₁₋₄Alkyl)-amino-C₁₋₃-alkylaminogruppe oder eine 4-bis 7-gliedrige Cycloalkyleniminogruppe, wobei der Cycloalkylenteil mit einem Phenylring kondensiert sein kann oder jeweils die Methylengruppe in Position 4 einer 6- oder 7-gliedrigen Cycloalkyleniminogruppe durch ein Sauerstoff- oder Schwefelatom, durch eine Sulfinyl-, Sulfonyl-, -NH-, -N(C₁₋₃-Alkyl)-, -N(Phenyl)-, -N(C₁₋₃-Alkyl-carbonyl)- oder -N(Benzoyl)-Gruppe ersetzt sein kann, bedeuten,
   eine C₄₋₇-Cycloalkylamino-, C₄₋₇-Cycloalkyl-C₁₋₃-alkylamino- oder C₄₋₇-Cycloalkenylaminogruppe, in der die Position 1 des Rings nicht an der Doppelbindung beteiligt ist und wobei die vorstehend genannten Gruppen jeweils zusätzlich am Aminstickstoffatom durch eine C₅₋₇-Cycloalkyl-, C₂₋₄-Alkenyl- oder C₁₋₄-Alkylgruppe substituiert sein können,
   oder eine 4- bis 7-gliedrige Cycloalkyleniminogruppe, in der
   der Cycloalkylenteil mit einer Phenylgruppe oder mit einer gegebenenfalls durch ein Fluor-, Chlor-, Brom- oder Iodatom, durch eine Nitro-, C₁₋₃-Alkyl-, C₁₋₃-Alkoxy- oder Aminogruppe substituierten Oxazolo-, Imidazolo-, Thiazolo-, Pyridino-, Pyrazino- oder Pyrimidinogruppe kondensiert sein kann oder/und
   ein oder zwei Wasserstoffatome jeweils durch eine C₁₋₃-Alkyl-, C₅₋₇-Cycloalkyl- oder Phenylgruppe ersetzt sein können oder/und
   die Methylengruppe in Position 3 einer 5-gliedrigen Cycloalkyleniminogruppe durch eine Hydroxy-, Hydroxy-C₁₋₃-alkyl-, C₁₋₃-Alkoxy- oder C₁₋₃-Alkoxy-C₁₋₃-alkylgruppe substituiert sein kann,
   jeweils die Methylengruppe in Position 3 oder 4 einer 6- oder 7-gliedrigen Cycloalkyleniminogruppe durch eine Hydroxy-, Hydroxy-C₁₋₃-alkyl-, C₁₋₃-Alkoxy-, C₁₋₃-Alkoxy-C₁₋₃-alkyl-, C₁₋₄-Alkoxycarbonyl-, Aminocarbonyl-, C₁₋₃-Alkylaminocarbonyl-, Di-(C₁₋₃-alkyl)-aminocarbonyl-, Phenyl-C₁₋₃-alkylamino- oder N-(C₁₋₃-Alkyl)-phenyl-C₁₋₃-alkylaminogruppe substituiert oder
   durch ein Sauerstoff- oder Schwefelatom, durch eine Sulfinyl-, Sulfonyl-, -NH-, -N(C₁₋₃-Alkyl-)-, -N(Phenyl)-, -N(Phenyl-C₁₋₃-alkyl-)-, -N(C₁₋₃-Alkyl-carbonyl-)-, -N(C₁₋₄-Alkoxy-carbonyl-)-, -N(Benzoyl-)- oder -N(Phenyl-C₁₋₃-alkyl-carbonyl-)-Gruppe ersetzt sein kann,
   wobei eine mit einem Imino-Stickstoffatom der Cycloalkyleniminogruppe verknüpfte Methylengruppe durch eine Carbonyl- oder Sulfonylgruppe ersetzt sein kann oder in einer 5- bis 7-gliedrigen monocyclischen oder mit einer Phenylgruppe kondensierten Cycloalkyleniminogruppe beide mit dem Imino-Stickstoffatom verknüpften Methylengruppen jeweils durch eine Carbonylgruppe ersetzt sein können,
   bedeutet:

Umsetzung einer Verbindung der allgemeinen Formel 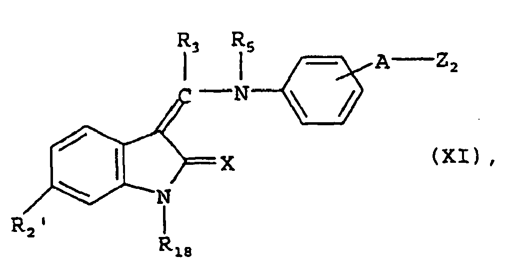 in der
R₃, R₅ und X wie eingangs erwähnt definiert sind,
R₂' die für R₂ eingangs erwähnten Bedeutungen besitzt,
R₁₈ ein Wasserstoffatom oder eine Schutzgruppe für das Stickstoffatom der Lactamgruppe, wobei einer der Reste R₂' und R₁₈ auch eine gegebenenenfalls über einen Spacer gebildete Bindung an eine Festphase darstellen kann und der andere der Reste R₂' und R₁₈ die vorstehend erwähnten Bedeutungen besitzt, A eine C₁₋₄-Alkylgruppe und Z₂ eine Austrittsgruppe, beispielsweise eine Alkyl- oder Arylsulfonyloxygruppe wie die Methylsulfonyloxy-, Ethylsulfonyloxy-, p-Toluolsulfonyloxy-, oder Trifluormethansulfonyloxygruppe darstellt, mit einem Amin der allgemeinen Formel

H—R_{7,} (XII),

in der
R₇ die vorstehend für R₇ genannten Bedeutungen besitzt, und erforderlichenfalls anschließende Abspaltung einer verwendeten Schutzgruppe für das Stickstoffatom der Lactamgruppe oder von einer Festphase.

Die Umsetzung wird zweckmäßigerweise in einem Lösungsmittel wie Methylenchlorid, Tetrahydrofuran, 1,4-Dioxan, Toluol, Acetonitril, Dimethylsulfoxid, Dimethylformamid, Dimethylacetamid, N-Methylpyrrolidon oder deren Gemischen, gegebenenfalls unter Zusatz von Wasser als Cosolvens oder/und unter Zusatz einer inerten Hilfsbase, beispielsweise Natriumhydrogencarbonat, Pyridin, 2,4,6-Trimethylpyridin, Chinolin, Triethylamin, N-Ethyldiisopropylamin, N-Ethyl-dicyclohexylamin, 1,4-Diazabicyclo[2,2,2]octan oder 1,8-Diazabicyclo[5,4,0]-undec-7-en, bei Temperaturen zwischen -50°C und +100°C, vorzugsweise zwischen -10°C und +50°C, durchgeführt, wobei eine verwendete Schutzgruppe infolge Umamidierung gleichzeitig abgespalten werden kann.

Die gegebenenfalls erforderliche Abspaltung einer verwendeten Schutzgruppe für das Stickstoffatom der Lactamgruppe oder von einer Festphase erfolgt wie vorstehend unter Verfahren (a) beschrieben.

Erhält man eine Verbindung der allgemeinen Formel I, die eine Alkoxycarbonylgruppe enthält, so kann diese mittels Hydrolyse in eine entsprechende Carboxyverbindung übergeführt werden, oder
eine Verbindung der allgemeinen Formel I, die eine Amino- oder Alkylaminogruppe enthält, so kann diese mittels reduktiver Alkylierung in eine entsprechende Alkylamino- oder Dialkylaminoverbindung übergeführt werden, oder
eine Verbindung der allgemeinen Formel I, die eine Amino- oder Alkylaminogruppe enthält, so kann diese mittels Acylierung oder Sulfonierung in eine entsprechende Acyl- oder Sulfonylverbindung übergeführt werden, oder
eine Verbindung der allgemeinen Formel I, die eine Carboxygruppe enthält, so kann diese mittels Veresterung oder Amidierung in eine entsprechende Ester- oder Aminocarbonylverbindung übergeführt werden, oder
eine Verbindung der allgemeinen Formel I, die eine Cycloalkyleniminogruppe enthält, in der eine Methylengruppe durch ein Schwefelatom ersetzt ist, so kann diese mittels Oxidation in eine entsprechende Sulfinyl- oder Sulfonylverbindung übergeführt werden, oder
eine Verbindung der allgemeinen Formel I, die eine Nitrogruppe enthält, so kann diese mittels Reduktion in eine entsprechende Aminoverbindung übergeführt werden, oder
eine Verbindung der allgemeinen Formel I, in der R₄ eine durch eine Amino-, Alkylamino-, Aminoalkyl- oder N-Alkyl-aminogruppe substituierte Phenylgruppe darstellt, so kann diese anschliessend mittels Umsetzung mit einem entsprechenden Cyanat, Isocyanat oder Carbamoylhalogenid in eine entsprechende Harnstoffverbindung der allgemeinen Formel I übergeführt werden oder
eine Verbindung der allgemeinen Formel I, in der R₄ eine durch eine Amino-, Alkylamino-, Aminoalkyl- oder N-Alkyl-aminogruppe substituierte Phenylgruppe darstellt, so kann diese anschliessend mittels Umsetzung mit einer entsprechenden die Amidinogruppe übertragenden Verbindung oder durch Umsetzung mit einem entsprechenden Nitril in eine entsprechende Guanidinoverbindung der allgemeinen Formel I übergeführt werden.

Die anschließende Hydrolyse erfolgt vorzugsweise in einem wäßrigen Lösungsmittel, z.B. in Wasser, Methanol/Wasser, Ethanol/ Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid bei Temperaturen zwischen 0 und 100°C, vorzugsweise bei Temperaturen zwischen 10 und 50°C.

Die anschließende reduktive Alkylierung wird vorzugsweise in einem geeigneten Lösungsmittel wie Methanol, Methanol/Wasser, Methanol/Wasser/Ammoniak, Ethanol, Ether, Tetrahydrofuran, Dioxan oder Dimethylformamid gegebenenfalls unter Zusatz einer Säure wie Salzsäure in Gegenwart von katalytisch angeregtem Wasserstoff, z.B. von Wasserstoff in Gegenwart von Raney-Nickel, Platin oder Palladium/Kohle, oder in Gegenwart eines Metallhydrids wie Natriumborhydrid, Lithiumborhydrid, Natriumcyanoborhydrid oder Lithiumaluminiumhydrid bei Temperaturen zwischen 0 und 100°C, vorzugsweise bei Temperaturen zwischen 20 und 80°C, durchgeführt.

Die anschließende Acylierung oder Sulfonylierung wird zweckmäßigerweise mit der entsprechenden freien Säure oder einer entsprechenden reaktionsfähigen Verbindung wie deren Anhydrid, Ester, Imidazolid oder Halogenid vorzugsweise in einem Lösungsmittel wie Methylenchlorid, Diethylether, Tetrahydrofuran, Toluol, Dioxan, Acetonitril, Dimethylsulfoxid oder Dimethylformamid gegebenenfalls in Gegenwart einer anorganischen oder einer tertiären organischen Base bei Temperaturen zwischen -20 und 200°C, vorzugsweise bei Temperaturen zwischen 20°C und der Siedetemperatur des verwendeten Lösungsmittel, durchgeführt. Die Umsetzung mit der freien Säure kann gegebenenfalls in Gegenwart eines die Säure aktivierenden Mittels oder eines wasserentziehenden Mittels, z.B. in Gegenwart von Chlorameisensäureisobutylester, Orthokohlensäuretetraethylester, Orthoessigsäuretrimethylester, 2,2-Dimethoxypropan, Tetramethoxysilan, Thionylchlorid, Trimethylchlorsilan, Phosphortrichlorid, Phosphorpentoxid, N,N'-Dicyclohexylcarbodiimid, N,N'-Dicyclohexylcarbodiimid/N-Hydroxysuccinimid, N,N'-Dicyclohexylcarbodiimid/1-Hydroxy-benztriazol, 2-(1H-Benzotriazol-1-yl)-1,1,3,3-tetramethyluronium-tetrafluorborat, 2-(1H-Benzotriazol-1-yl)-1,1,3,3-tetramethyluronium-tetrafluorborat/1-Hydroxy-benztriazol, N,N'-Carbonyldiimidazol oder Triphenylphosphin/Tetrachlorkohlenstoff, und gegebenenfalls unter Zusatz einer Base wie Pyridin, 4-Dimethylamino-pyridin, N-Methyl-morpholin oder Triethylamin zweckmäßigerweise bei Temperaturen zwischen 0 und 150°C, vorzugsweise bei Temperaturen zwischen 0 und 100°C, erfolgen. Die Umsetzung mit einer entsprechenden reaktionsfähigen Verbindung kann gegebenenfalls in Gegenwart einer tertiären organischen Base wie Triethylamin, N-Ethyl-diisopropylamin, N-Methyl-morpholin oder Pyridin oder bei Verwendung eines Anhydrids bei Gegenwart der entsprechenden Säure bei Temperaturen zwischen 0 und 150°C, vorzugsweise bei Temperaturen zwischen 50 und 100°C, erfolgen.

Die anschließende Veresterung oder Amidierung wird zweckmäßigerweise durch Umsetzung eines reaktionsfähigen entsprechenden Carbonsäurederivates mit einem entsprechenden Alkohol oder Amin wie vorstehend beschrieben durchgeführt.

Die anschließende Oxidation des Schwefelatoms wird vorzugsweise in einem Lösungsmittel oder Lösungsmittelgemisch, z.B. in Wasser, Wasser/Pyridin, Aceton, Methylenchlorid, Essigsäure, Essigsäure/Acetanhydrid, verdünnter Schwefelsäure oder Trifluoressigsäure, je nach dem verwendeten Oxidationsmittel zweckmäßigerweise bei Temperaturen zwischen -80 und 100°C durchgeführt. Zur Herstellung einer entsprechenden Sulfinylverbindung der allgemeinen Formel I wird die Oxidation zweckmäßigerweise mit einem Äquivalent des verwendeten Oxidationsmittels durchgeführt, z.B. mit Wasserstoffperoxid in Eisessig, Trifluoressigsäure oder Ameisensäure bei 0 bis 20°C oder in Aceton bei 0 bis 60°C, mit einer Persäure wie Perameisensäure in Eisessig oder Trifluoressigsäure bei 0 bis 50°C oder mit m-Chlorperbenzoesäure in Methylenchlorid, Chloroform oder Dioxan bei -20 bis 80°C, mit Natriummetaperjodat in wäßrigem Methanol oder Ethanol bei -15 bis 25°C, mit Brom in Eisessig oder wäßriger Essigsäure gegebenenfalls in Gegenwart einer schwachen Base wie Natriumacetat, mit N-Bromsuccinimid in Ethanol, mit tert.-Butylhypochlorit in Methanol bei -80 bis -30°C, mit Iodbenzodichlorid in wäßrigem Pyridin bei 0 bis 50°C, mit Salpetersäure in Eisessig bei 0 bis 20°C, mit Chromsäure in Eisessig oder in Aceton bei 0 bis 20°C und mit Sulfurylchlorid in Methylenchlorid bei -70°C, der hierbei erhaltene Thioether-Chlor-Komplex wird zweckmäßigerweise mit wäßrigem Ethanol hydrolysiert.

Zur Herstellung einer Sulfonylverbindung der allgemeinen Formel I wird die Oxidation ausgehend von einer entsprechenden Sulfinylverbindung zweckmäßigerweise mit einem oder mehr Äquivalenten des verwendeten Oxidationsmittels oder ausgehend von einer entsprechenden Mercaptoverbindung zweckmäßigerweise mit zwei oder mehr Äquivalenten des verwendeten Oxidationsmittels durchgeführt, z.B. mit Wasserstoffperoxid in Eisessig/Acetanhydrid, Trifluoressigsäure oder in Ameisensäure bei 20 bis 100°C oder in Aceton bei 0 bis 60°C, mit einer Persäure wie Perameisensäure oder m-Chlorperbenzoesäure in Eisessig, Trifluoressigsäure, Methylenchlorid oder Chloroform bei Temperaturen zwischen 0 und 60°C, mit Salpetersäure in Eisessig bei 0 bis 20°C, mit Chromsäure, Natriumperjodat oder Kaliumpermanganat in Essigsäure, Wasser/Schwefelsäure oder in Aceton bei 0 bis 20°C.

Die anschließende Reduktion einer Nitrogruppe erfolgt vorzugsweise hydrogenolytisch, z.B. mit Wasserstoff in Gegenwart eines Katalysators wie Palladium/Kohle oder Raney-Nickel in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure oder Eisessig bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die anschließende Herstellung einer entsprechende Harnstoffverbindung der allgemeinen Formel I wird zweckmäßigerweise mit einem anorganischen Cyanat oder einem entsprechenden Isocyanat oder Carbamoylchlorid vorzugsweise in einem Lösungsmittel wie Dimethylformamid und gegebenenfalls in Gegenwart einer tertiären organischen Base wie Triethylamin bei Temperaturen zwischen 0 und 50°C, vorzugsweise bei der Raumtemperatur, durchgeführt.

Die anschließende Herstellung einer entsprechenden Guanidinoverbindung der allgemeinen Formel I wird zweckmäßigerweise durch Umsetzung mit einer die Amidinogruppe übertragenden Verbindung wie 3,5-Dimethylpyrazol-1-carbonsäureamidin vorzugsweise in einem Lösungsmittel wie Dimethylformamid und gegebenenfalls in Gegenwart einer tertiären organischen Base wie Triethylamin bei Temperaturen zwischen 0 und 50°C, vorzugsweise bei der Raumtemperatur, durchgeführt.

Bei den vorstehend beschriebenen Umsetzungen können gegebenenfalls vorhandene reaktive Gruppen wie Carboxy-, Hydroxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Carboxygruppe die Trimethylsilyl-, Methyl-, Ethyl-, tert.Butyl-, Benzyl- oder Tetrahydropyranylgruppe und
als Schutzrest für eine Hydroxy-, Amino-, Alkylamino- oder Iminogruppe die Acetyl-, Trifluoracetyl-, Benzoyl-, Ethoxycarbonyl-, tert.Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure oder Eisessig bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen.

Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan, Essigester oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Ferner können erhaltene chirale Verbindungen der allgemeinen Formel I in ihre Enantiomeren und/oder Diastereomeren aufgetrennt werden.

So lassen sich beispielsweise die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes 2 asymmetrischen Kohlenstoffatomen auf Grund ihrer physikalisch-chemischen Unterschiede nach an sich bekannten Methoden, z.B. durch Chromatographie und/- oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen Gemisches diastereomerer Salze oder Derivate, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure, Dibenzoylweinsäure, Di-o-Tolylweinsäure, Apfelsäure, Mandelsäure, Camphersulfonsäure, Glutaminsäure, N-Acetylglutaminsäure, Asparaginsäure, N-Acetyl-asparaginsäure oder Chinasäure. Als optisch aktiver Alkohol kommt beispielsweise (+)- oder (-)-Menthol und als optisch aktiver Acylrest in Amiden beispielsweise der (+)- oder (-)-Menthyloxycarbonylrest in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure, Maleinsäure oder Methansulfonsäure in Betracht.

Außerdem lassen sich die so erhaltenen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Die als Ausgangsprodukte verwendeten Verbindungen der allgemeinen Formeln VII bis XII sind teilweise literaturbekannt oder man erhält diese nach literaturbekannten Verfahren oder können nach den vorstehend und in den Beispielen beschriebenen Verfahren erhalten werden. Beispielsweise werden die Verbindungen der allgemeinen Formel IX in der deutschen Patentanmeldung 198 24 922.5 beschrieben. Ferner sind die Verbindungen der allgemeinen XI aus den Verbindungen der allgemeinen Formel I, in denen R₄ eine im Alkylteil durch eine Hydroxygruppe substituierte C₁₋₄-Alkyl-phenylgruppe darstellt, beispielsweise durch Umsetzung mit Alkyl- oder Arylsulfonylchloriden zugänglich.

Wie bereits eingangs erwähnt, weisen die Verbindungen der allgemeinen Formel I, in der R₁ ein Wasserstoffatom oder einen Prodrugrest darstellt, wertvolle pharmakologische Eigenschaften auf, insbesondere eine inhibierende Wirkung auf verschiedene Kinasen, vor allem auf Rezeptor-Tyrosinkinasen wie VEGFR2, , PDGFRα, PDGFRβ, FGFR1, FGFR3, EGFR, HER2, IGF1R und HGFR, sowie auf Komplexe von CDK's (Cyclin Dependent Kinases) wie CDK1, CDK2, CDK3, CDK4, CDK5, CDK6, CDK7, CDK8 und CDK9 mit ihren spezifischen Cyclinen (A, B1, B2, C, D1, D2, D3, E, F, G1, G2, H, I und K) und auf virales Cyclin, auf die Proliferation kultivierter humaner Zellen, insbesondere die von Endothelzellen, z.B. bei der Angiogenese, aber auch auf die Proliferation anderer Zellen, insbesondere von Tumorzellen.

Die biologischen Eigenschaften der Verbindungen der allgemeinen Formel (I), bezeichungsweise der erfindungsgemäß Verbindung, wurde nach folgendem Standardverfahren wie folgt geprüft:

Humane Nabelschnur Endothelzellen (HUVEC) wurden in IMDM (Gibco BRL), supplementiert mit 10 % foetalem Rinderserum (FBS) (Sigma), 50 µM ß-Mercaptoeethanol (Fluka), Standardantibiotika, 15 µg/ml Endothelzellwachstumsfaktor (ECGS, Collaborative Biomedical Products) und 100 µg/ml Heparin (Sigma) auf Gelatine-beschichteten Kulturflaschen (0.2 % Gelatine, Sigma) bei 37°C, 5 % CO₂ in wassergesättigter Atmosphäre kultiviert.

Zur Untersuchung der inhibitorischen Aktivität der Verbindungen der allgemeinen Formel (I), bezeichungsweise der erfindungsgemäß Verbindung, wurden die Zellen für 16 Stunden "gehungerbt", d.h. in Kulturmedium ohne Wachstumsfaktoren (ECGS + Heparin) gehalten. Die Zellen wurden mittels Trypsin/EDTA von den Kulturflaschen abgelöst und einmal in serumhaltigem Medium gewaschen. Anschließend wurden 2,5 x 10³ Zellen pro well ausgesät.

Die Proliferation der Zellen wurde mit 5 ng/ml VEGF₁₆₅ (vascular endothelial growth factor; H. Weich, GBF Braunschweig) und 10 µg/ml Heparin stimuliert. Pro Platte wurden jeweils 6 wells als Kontrollwert nicht stimuliert.

Die Verbindungen wurden in 100 % Dimethylsulfoxid gelöst und in verschiedenen Verdünnungen als Dreifachbestimmungen den Kulturen zugefügt, wobei die maximale Dimethylsulfoxid-Konzentration 0.3 % betrug.

Die Zellen wurden für 76 Stunden bei 37°C inkubiert, dann wurde für weitere 16 Stunden ³H-Thymidin (0.1 µ Ci/well, Amersham) zugegeben, um die DNA Synthese zu bestimmen. Anschließend wurden die radioaktiv markierten Zellen auf Filtermatten immobilisiert und die eingebaute Radioaktivität in einem βcounter bestimmt. Zur Bestimmung der inhibitorischen Aktivität der Verbindungen wurde der Mittelwert der nicht-stimulierten Zellen vom Mittelwert der Faktor-stimulierten Zellen (in Anwesenheit oder Abwesenheit der Verbindungen) subtrahiert.

Die relative Zellproliferation wurde in Prozent der Kontrolle (HUVEC ohne Inhibitor) berechnet und die Wirkstoffkonzentration, die die Proliferation der Zellen zu 50 % hemmt (IC₅₀), abgeleitet.

Auf Grund ihrer Hemmwirkung auf die Proliferation von Zellen, insbesondere von Endothelzellen und von Tumorzellen, eignen sich die Verbindungen der allgemeinen Formel I, bezeichungsweise die erfindungsgemäße Verbindung, zur Behandlung von Krankheiten, in denen die Proliferation von Zellen, insbesondere die von Endothelzellen, eine Rolle spielt.

So stellt beispielsweise die Proliferation von Endothelzellen und die damit verbundene Neovaskularisierung einen entscheidenden Schritt bei der Tumorprogression dar (Folkman J. et al., Nature 339, 58-61, (1989); Hanahan D. und Folkman J., Cell 86, 353-365, (1996)). Weiterhin ist die Proliferation von Endothelzellen auch bei Hämangiomen, bei der Metastasierung, der rheumatischen Arthritis, der Psoriasis und der okularen Neovaskularisierung von Bedeutung (Folkman J., Nature Med. 1, 27-31, (1995)). Der therapeutische Nutzen von Inhibitoren der Endothelzellproliferation wurde im Tiermodell beispielsweise von O'Reilly et al. und Parangi et al. gezeigt (O'Reilly M.S. et al., Cell 88, 277-285, (1997); Parangi S. et al., Proc Natl Acad Sci USA 93, 2002-2007, (1996)).

Die Verbindungen der allgemeinen Formel I, bezeichungsweise die erfindungsgemäße Verbindung, deren Tautomeren, deren Stereoisomere oder deren physiologisch verträglichen Salze eignen sich somit beispielsweise zur Behandlung von Tumoren (z. B. Plattenepithelkarzinom, Astrozytom, Kaposi's Sarkom, Glioblastom, Lungenkrebs, Blasenkrebs, Hals- und Nackenkarzimom, Melanom, Ovarkarzinom, Prostatakarzinom, Brustkrebs, kleinzelliges Lungenkarzinom, Gliom, Colorektalkarzinom, urogenital Krebs und gastrointestinal Karzinom sowie hämatologischer Krebserkrankungen, wie multiples Myelom), Psoriasis, Arthritis (z. B. rheumatoide Arthritis), Hämangioma, Angiofibroma, Augenerkrankungen (z.B. diabetische Retinopathie), neovaskulares Glaukom, Nierenerkrankungen (z.B. Glomerulonephritis), diabetische Nephropathie, maligne Nephrosklerose, thrombische mikroangiopathische Syndrome, Transplantationsabstossungen und Glomerulopathie, fibrotische Erkrankungen (z. B. Leberzirrhose), mesangialzellproliferative Erkrankungen, Artheriosklerose, Verletzungen des Nervengewebes und zur Hemmung der Reocclusion von Gefässen nach Ballonkatheterbehandlung, bei der Gefässprothetik oder nach dem Einsetzen von mechanischen Vorrichtungen zum Offenhalten von Gefässen (z.B. Stents), oder anderen Erkrankungen, bei denen Zellproliferation oder Angiogenese eine Rolle spielen.

Auf Grund ihrer biologischen Eigenschaften können die verbindungen, bezeichnungsweise die erfindungsgemäße Verbindung, allein oder in Kombination mit anderen pharmakologisch wirksamen Verbindungen angewendet werden, beispielsweise in der Tumortherapie in Monotherapie oder in Kombination mit anderen Anti-Tumor Therapeutika, beispielsweise in Kombination mit Topoisomerase-Inhibitoren (z.B. Etoposide), Mitoseinhibitoren (z.B. Vinblastin, Taxol), mit Nukleinsäuren interagierenden Verbindungen (z.B. cis-Platin, Cyclophosphamid, Adriamycin), Hormon-Antagonisten (z.B. Tamoxifen), Inhibitoren metabolischer Prozesse (z.B. 5-FU etc.), Zytokinen (z.B. Interferonen), Kinase-Inhibitoren, Antikörpern, oder auch in Kombination mit Strahlentherapie etc. Diese Kombinationen können entweder simultan oder sequentiell verabreicht werden.

Bei der pharmazeutischen Anwendung werden die Verbindungen in der Regel bei warmblütigen Wirbeltieren, insbesondere beim Menschen, in Dosierungen von 0,01-100 mg/kg Körpergewicht, vorzugsweise bei 0,1-20 mg/kg verwendet. Zur Verabreichung werden diese mit einem oder mehreren üblichen inerten Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/- Sorbit, Wasser/Polyäthylenglykol, Propylenglykol, Stearylalkohol, Carboxymethylcellulose oder fetthaltigen Substanzen wie Hartfett oder deren geeigneten Gemischen in übliche galenische Zubereitungen wie Tabletten, Dragées, Kapseln, Pulver, Injektionslösungen, Ampullen, Suspensionen, Lösungen, Sprays oder Zäpfchen eingearbeitet.

Die nachfolgenden Beispiele sollen die Erfindung näher erläutern:

### Verwendete Abkürzungen:

FMOC = 9-Fluorenylmethoxycarbonyl
HOBt = 1-Hydroxy-1H-benzotriazol
TBTU = O-Benzotriazol-1-yl-N,N,N',N'-tetramethyluronium-tetrafluoroborat
DBU = 1,8-Diazabicyclo[5.4.0]undec-7-en

### Herstellung der Ausgangsverbindungen:

### Festphasenbeispiel T

2.0 g Rink-Harz (MBHA-Harz, Firma Novabiochem) läßt man in 30 ml Dimethylformamid quellen. Anschließend gibt man 40 ml 30%iges Piperidin in Dimethylformamid zu und schüttelt 7 Minuten, um die FMOC-Schutzgruppe abzuspalten. Dann wird das Harz mehrmals mit Dimethylformamid gewaschen. Anschließend gibt man 0.4 g 2-Indolinon-6-carbonsäure (Herstellung analog Langenbeck et al., Justus Liebigs Ann. Chem. 499, 201-208 (1932)), 297 mg HOBt, 706 mg TBTU und 0.9 ml N-Ethyl-diisopropylamin in 30 ml Dimethylformamid zu und schüttelt 1 Stunde. Dann wird die Lösung abgesaugt und das Harz fünfmal mit 30 ml Dimethylformamid und dreimal mit 30 ml Methylenchlorid gewaschen. Zum Trocknen wird Stickstoff durch das Harz geblasen.
Ausbeute: 1.9 g beladenes Harz

### Festphasenbeispiel II

1.9 g des gemäß Beispiel I erhaltenen Harzes werden mit 6 ml Acetanhydrid und 6 ml Orthobenzoesäuretriethylester 3 Stunden bei 110°C gerührt. Danach läßt man abkühlen und wäscht das Harz mit Dimethylformamid und anschließend mit Methylenchlorid.
Ausbeute: 1.9 g feuchtes Harz

Analog Beispiel II werden folgende beladene Harze hergestellt:
(1) Mit 3-z-(1-Ethoxy-methylen)-6-carbamoyl-2-indolinon belegtes Harz
   Hergestellt durch Umsetzung des gemäß Beispiel I erhaltenen Harzes mit Orthoameisensäuretriethylester
(2) Mit 3-z-(1-Methoxy-1-methyl-methylen)-6-carbamoyl-2-indolinon belegtes Harz
   Hergestellt durch Umsetzung des gemäß Beispiel I erhaltenen Harzes mit Orthoessigsäuretrimethylester
(3) Mit 3-z-(1-Methoxy-1-ethyl-methylen)-6-carbamoyl-2-indolinon belegtes Harz
   Hergestellt durch Umsetzung des gemäß Beispiel I erhaltenen Harzes mit Orthopropionsäuretrimethylester
(4) Mit 3-z-(1-Methoxy-1-propyl-methylen)-6-carbamoyl-2-indolinon belegtes Harz
   Hergestellt durch Umsetzung des Produktes aus Beispiel I und Orthobuttersäuretrimethylester

### Beispiel III

### N-(4-Nitrophenyl)-N-methyl-methansulfonamid

3.0 g N-Methyl-4-nitroanilin werden in 20 ml Pyridin gelöst und 2.4 g Methansulfonsäurechlorid bei Raumtemperatur zugetropft. Das Gemisch wird für 12 Stunden bei Raumtemperatur gerührt. Nach dieser Zeit wird das Gemisch auf Wasser gegossen, der ausgefallenen Niederschlag abfiltriert und bei 50°C im Vakuum getrocknet.
Ausbeute: 4.0 g (87 % der Theorie),
R_{f}-Wert: 0.5 (Kieselgel, Essigester/Toluol = 7:3)
Schmelzpunkt: 107-108 °C

### Beispiel IV

### N-(2-Dimethylamino-ethyl)-N-methylsulfonyl-4-nitroanilin

38.9 g N-Methylsulfonyl-4-nitroanilin werden in 2.0 1 Aceton gelöst, 51.9 g 1-Chlor-2-dimethylamino-ethan, 77.4 g Kaliumcarbonat und 5.0 g Natriumiodid zugesetzt und das Gemisch insgesamt 4 Tage bei 50°C gerührt, wobei nach 12 Stunden weitere 25.9 g 1-Chlor-2-dimethylamino-ethan, 49.8 g Kaliumcarbonat und 5.0 g Natriumiodid in 500 ml Aceton und nach 36 Stunden weitere 26.0 g 1-Chlor-2-dimethylamino-ethan, 50.0 g Kaliumcarbonat und 5.0 g Natriumiodid in 100 ml Aceton zugesetzt werden. Nach dieser Zeit wird der Ansatz filtriert und das Filtrat eingeengt. Der Rückstand wird mit Ether verrührt, abgesaugt und bei 40°C getrocknet.
Ausbeute: 25.3 g (49 % der Theorie),
R_{f}-Wert: 0.5 (Kieselgel, Methylenchlorid/Methanol/Ammoniak = 9:1:0.1)
C₁₁H₁₇N₃O₄S
ESI-Massenspektrum: m/z = 288 [M+H⁺]

Analog Beispiel IV werden folgende Verbindungen hergestellt:
(1) 4-[N-(3-Dimethylamino-propyl)-N-methylsulfonyl-amino]-nitrobenzol
(2) N-Carboxymethyl-N-methylsulfonyl-4-nitroanilin
(3) N-Cyanomethyl-N-methylsulfonyl-p-phenylendiamin
(4) 4-[N-(2-(N-Benzyl-N-methyl-amino)-ethyl)-N-methylsulfonylamino]-nitrobenzol
(5) 4-[N-(3-Phthalimido-2-yl-propyl)-N-methylsulfonyl-amino]-nitrobenzol
(6) 4-[N-(3-(N-Benzyl-N-methyl-amino)-propyl)-N-methylsulfonyl-aminol-nitrobenzol

### Beispiel V

### N-(Dimethylaminocarbonyl-methyl)-N-methylsulfonyl-4-nitroanilin

7.0 g N-Carboxymethyl-N-methylsulfonyl-4-nitroanilin, 2.5 g Dimethylaminhydrochlorid, 8.1 g TBTU und 3.9 g HOBT werden in 125 ml Dimethylformamid gelöst und bei 0°C 17.6 ml N-Ethyl-diisopropylamin zugegeben. Das Gemisch wird 4 Stunden bei Raumtemperatur gerührt, mit 1 1 Wasser verdünnt und der ausgefallene Niederschlag abgesaugt. Nach Waschen mit Wasser, Ethanol und Ether wird der Rückstand bei 70°C im Vakuum getrocknet.
Ausbeute: 5.3 g (69 % der Theorie),
R_{f}-Wert: 0.40 (Kieselgel, Methylenchlorid/Methanol = 9:1) C₁₁H₁₅N₃O₅S
ESI-Massenspektrum: m/z = 300 [M-H⁻]

Analog Beispiel V werden folgende Verbindungen hergestellt:
(1) 4-[(N-Dimethylaminocarbonylmethyl)-amino]-nitrobenzol Hergestellt aus 4-(N-Carboxymethyl-amino)-nitrobenzol und Dimethylaminhydrochlorid
(2) 4-(N-Methylaminocarbonylmethyl-N-methylsulfonyl-amino)-nitrobenzol
   Hergestellt aus N-Carboxymethyl-N-methylsulfonyl-4-nitroanilin und Methylaminhydrochlorid
(3) 4-[(N-(Methylcarbamoyl-methyl)-N-methyl-amino)-methyl]-nitrobenzol
   Hergestellt aus 4-[(N-Carboxymethyl-N-methyl-amino)-methyl]-nitrobenzol und Methylaminhydrochlorid
(4) 4-[(N-(Dimethylcarbamoyl-methyl)-N-methyl-amino)-methyl]-nitrobenzol
   Hergestellt aus 4-[(N-Carboxymethyl-N-methyl-amino)-methyl]-nitrobenzol und Dimethylaminhydrochlorid

### Beispiel VI

### 4-[N-(2-Dimethylamino-ethyl)-N-acetyl-amino]-nitrobenzol

3.6 g 4-(2-Dimethylamino-ethylamino)-nitrobenzol (nach Gabbay et al., J. Am. Chem. Soc. 91, 5136 (1969)) werden in 50 ml Methylenchlorid gelöst und 5.0 ml Triethylamin zugegeben. Zu dieser Mischung werden langsam 1.3 ml Acetylchlorid bei Raumtemperatur zugetropft und das Gemisch 2 Stunden bei Raumtemperatur gerührt. Nach dieser Zeit werden nochmals 5.0 ml Triethylamin und 1.3 ml Acetylchlorid zugegeben und weitere 2 Stunden am Rückfluß gekocht. Das Lösungsmittel wird abgezogen, der Rückstand in Essigester aufgenommen und die organische Phase zweimal mit Wasser ausgeschüttelt. Nach Trocknen über MgSO₄ wird das Lösungsmittel abgezogen und der Rückstand im Vakuum getrocknet.
Ausbeute: 2.0 g (45 % der Theorie),
R_{f}-Wert: 0.55 (Kieselgel, Methylenchlorid/Methanol/Ammoniak = 9:1:0.1)
C₁₂H₁₇N₃O₃
ESI-Massenspektrum: m/z = 252 [M+H⁺]

Analog Beispiel VI werden folgende Verbindungen hergestellt:
(1) 4-[N-(3-Dimethylamino-propyl)-N-acetyl-amino]-nitrobenzol Hergestellt aus 4-(3-Dimethylamino-propylamino)-nitrobenzol (nach Gabbay et al., J. Am. Chem. Soc. 91, 5136 (1969) und Acetylchlorid
(2) 4-[N-(2-Dimethylamino-ethyl)-N-propionyl-amino]-nitrobenzol
   Hergestellt aus 4-(2-Dimethylamino-ethylamino)-nitrobenzol und Propionylchlorid
(3) 4-[N-Acetyl-N-(dimethylaminocarbonylmethyl)-amino]-nitrobenzol
   Hergestellt aus 4-[N-(Dimethylaminocarbonylmethyl)-amino]-nitrobenzol und Acetylchlorid
(4) 4-[N-(2-Dimethylamino-ethyl)-N-butyryl-amino]-nitrobenzol Hergestellt aus 4-(2-Dimethylamino-ethylamino)-nitrobenzol und Butyrylchlorid
(5) 4-[N-(2-Dimethylamino-ethyl)-N-isobutyryl-amino]-nitrobenzol
   Hergestellt aus 4-(2-Dimethylamino-ethylamino)-nitrobenzol und Isobutyrylchlorid
(6) 4-[N-(2-Dimethylamino-ethyl)-N-benzoyl-amino]-nitrobenzol Hergestellt aus 4-(2-Dimethylamino-ethylamino)-nitrobenzol und Benzoylchlorid
(7) 4-[N-(2-Dimethylamino-ethyl)-N-acetyl-amino]-1,3-dinitrobenzol
   Hergestellt aus 4-(2-Dimethylamino-ethyl-amino)-1,3-dinitrobenzol und Acetylchlorid
(8) 4-[N-(2-Dimethylamino-ethyl)-N-(furan-2-carbonyl)-amino]-nitrobenzol
   Hergestellt aus 4-(2-Dimethylamino-ethylamino)-nitrobenzol und Furan-2-carbonylchlorid
(9) 4-[N-(2-Dimethylamino-ethyl)-N-(2-methoxy-benzoyl)-amino]-nitrobenzol
   Hergestellt aus 4-(2-Dimethylamino-ethylamino)-nitrobenzol und 2-Methoxy-benzoylchlorid
(10) 4-[N-(2-Dimethylamino-ethyl)-N-(pyridin-3-carbonyl)-amino]-nitrobenzol
   Hergestellt aus 4-(2-Dimethylamino-ethylamino)-nitrobenzol und Nicotinsäurechlorid
(11) 4-[N-(2-Dimethylamino-ethyl)-N-(phenyl-acetyl)-amino]-nitrobenzol
   Hergestellt aus 4-(2-Dimethylamino-ethylamino)-nitrobenzol und Phenylacetyl-chlorid
(12) 4-[N-(2-Dimethylamino-ethyl)-N-acetyl-amino]-3-bromnitrobenzol
   Hergestellt aus 4-[N-(2-Dimethylamino-ethyl)-amino]-3-bromnitrobenzol und Acetylchlorid
(13) N-Acryloyl-N-methyl-4-nitro-anilin
   Hergestellt aus 4-Methylamino-nitrobenzol und Acrylsäurechlorid
(14) N-Acryloyl-N-isopropyl-4-nitro-anilin
   Hergestellt aus 4-Isopropylamino-nitrobenzol und Acrylsäurechlorid
(15) N-Acryloyl-N-benzyl-4-nitro-anilin
   Hergestellt aus 4-Benzylamino-nitrobenzol und Acrylsäurechlorid
(16) N-Bromacetyl-N-methyl-4-nitro-anilin
   Hergestellt aus 4-Methylamino-nitrobenzol und Bromacetylchlorid
(17) N-Bromacetyl-N-isopropyl-4-nitro-anilin
   Hergestellt aus 4-Isopropylamino-nitrobenzol und Bromacetylchlorid
(18) N-Bromacetyl-N-benzyl-4-nitro-anilin
   Hergestellt aus 4-Benzylamino-nitrobenzol und Bromacetylchlorid

### Beispiel VII

### N-(Dimethylaminomethylcarbonyl)-N-methyl-4-nitro-anilin

1.8 g Dimethylaminhydrochlorid und 5.5 g Kaliumcarbonat werden in 80 ml Aceton vorgelegt und 4.2 g N-Bromacetyl-N-methyl-4-nitroanilin in drei Portionen bei Raumtemperatur zugegeben. Das Gemisch wird für 12 Stunden bei Raumtemperatur gerührt. Nach dieser Zeit wird das Gemisch filtriert und das Filtrat eingeengt. Der Rückstand wird in Essigester gelöst, zweimal mit Wasser gewaschen, über Natriumsulfat getrocknet und schließlich einrotiert.
Ausbeute: 2.8 g (79 % der Theorie),
R_{f}-Wert: 0.5 (Kieselgel, Essigester/Methanol = 7:3) Schmelzpunkt: 121-122°C

Analog Beispiel VII werden folgende Verbindungen hergestellt:
(1) N-(Piperidin-1-yl-methylcarbonyl)-N-methyl-4-nitroanilin
(2) N-(Morpholin-4-yl-methylcarbonyl)-N-methyl-4-nitroanilin
(3) N-[(4-Benzyl-piperazin-1-yl)-methylcarbonyl]-N-methyl-4-nitroanilin
(4) N-(Pyrrolidin-1-yl-methylcarbonyl)-N-methyl-4-nitroanilin
(5) N-[(N-Aminocarbonylmethyl-N-methyl-amino)-methylcarbonyl]-N-methyl-4-nitroanilin
(6) N-[(N-Benzyl-N-methyl-amino)-methylcarbonyl]-N-methyl-4-nitroanilin
(7) N-[Di-(2-methoxyethyl)-amino-methylcarbonyl]-N-methyl-4-nitroanilin
(8) N-(Dimethylaminomethylcarbonyl)-N-isopropyl-4-nitro-anilin
(9) N-(Piperidin-1-yl-methylcarbonyl)-N-isopropyl-4-nitroanilin
(10) N-[(4-tert.Butoxycarbonyl-piperazin-1-yl)-methylcarbonyl]-N-isopropyl-4-nitro-anilin
(11) N-[(N-Benzyl-N-methyl-amino)-methylcarbonyl]-N-benzyl-4-nitro-anilin
(12) N-(Dimethylaminomethylcarbonyl)-N-benzyl-4-nitro-anilin
(13) N-(Piperidin-1-yl-methylcarbonyl)-N-benzyl-4-nitro-anilin
(14) N-[Di-(2-hydroxyethyl)-amino-methylcarbonyl]-N-methyl-4-nitroanilin
(15) N-[(N-(2-Methoxyethyl)-N-methyl-amino)-methylcarbonyl]-N-methyl-4-nitroanilin
(16) N-[(N-(2-Dimethylamino-ethyl)-N-methyl-amino)-methylcarbonyl]-N-methyl-4-nitroanilin
(17) N-[(4-Methyl-piperazin-1-yl)-methylcarbonyl]-N-methyl-4-nitroanilin
(18) N-[(Imidazol-1-yl)-methylcarbonyl]-N-methyl-4-nitroanilin
(19) N-[(Phthalimido-2-yl)-methylcarbonyl]-N-methyl-4-nitroanilin

### Beispiel VIII

### N-[(2-Dimethylamino-ethyl)-carbonyl]-N-benzyl-4-nitro-anilin

0.5 g Dimethylaminhydrochlorid, 1.1 ml Triethylamin und 1.2 g N-Acryloyl-N-benzyl-4-nitro-anilin werden in 50 ml Methanol gelöst und für 24 Stunden bei Raumtemperatur gerührt. Nach dieser Zeit wird das Gemisch eingeengt. Der Rückstand wird über eine Aluminiumoxidsäule (Aktivität 2-3) mit Methylenchlorid/Ethanol 50:1 als Laufmittel aufgereinigt.
Ausbeute: 1.4 g (98 % der Theorie),
R_{f}-Wert: 0.8 (Aluminiumoxid, Methylenchlorid/Ethanol = 20:1) Schmelzpunkt: 73°C

Analog Beispiel VIII werden folgende Verbindungen hergestellt:
(1) N-[(2-Dimethylamino-ethyl)-carbonyl]-N-isopropyl-4-nitro-anilin
   Hergestellt aus N-Acryloyl-N-isopropyl-4-nitro-anilin und Dimethylaminhydrochlorid
(2) N-[(2-Dimethylamino-ethyl)-carbonyl]-N-methyl-4-nitro-anilin
   Hergestellt aus N-Acryloyl-N-methyl-4-nitro-anilin und Dimethylaminhydrochlorid
(3) N-[(2-(4-tert.Butoxycarbonyl-piperazin-1-yl)-ethyl)-carbonyl]-N-methyl-4-nitro-anilin
   Hergestellt aus N-Acryloyl-N-methyl-4-nitro-anilin und N-tert.Butoxycarbonyl-piperazin
(4) N-[(2-(Piperidin-1-yl)-ethyl)-carbonyl]-N-methyl-4-nitro-anilin
   Hergestellt aus N-Acryloyl-N-methyl-4-nitro-anilin und Piperidin
(5) N-[(2-(N-Benzyl-N-methyl-amino)-ethyl)-carbonyl]-N-methyl-4-nitro-anilin
   Hergestellt aus N-Acryloyl-N-methyl-4-nitro-anilin und N-Benzyl-N-methyl-amin

### Beispiel IX

### 4-(4-Methyl-piperazin-1-yl)-nitrobenzol

31.5 g 4-Chlor-1-nitrobenzol und 44.4 ml 1-Methylpiperazin werden zusammengegeben und 18 Stunden bei 90°C gerührt. Anschließend wird die Lösung auf Eiswasser gegossen und der ausgefallene Niederschlag abgesaugt, mit Wasser gewaschen und aus Ethanol/Wasser 1:1 umkristallisiert. Der Rückstand wird im Vakuum bei 75°C getrocknet.
Ausbeute: 44.0 g (99 % der Theorie),
R_{f}-Wert: 0.5 (Kieselgel, Methylenchlorid/Methanol = 10:1) Schmelzpunkt: 108-112°C

Analog Beispiel IX werden folgende Verbindungen hergestellt:
(1) N-(2-Dimethylaminoethyl)-N-methyl-4-nitroanilin Hergestellt aus 1-Fluor-4-nitrobenzol und 1-Dimethylamino-2-methylamino-ethan
(2) N-(3-Dimethylaminopropyl)-N-methyl-4-nitroanilin Hergestellt aus 1-Fluor-4-nitrobenzol und 1-Dimethylamino-3-methylamino-propan
(3) 4-(N-Carboxymethyl-amino)-nitrobenzol
   Hergestellt aus 1-Fluor-4-nitrobenzol und Glycin
(4) N-Cyclohexyl-p-phenylendiamin
   Hergestellt aus 1-Fluor-4-nitrobenzol und Cyclohexylamin
(5) 6-[N-(2-Dimethylamino-ethyl)-N-methylsulfonyl-amino]-3-phthalimido-2-yl-nitrobenzol
   Hergestellt aus 2-Nitro-4-phthalimido-2-yl-fluorbenzol, N-(2-Dimethylamino-ethyl)-methansulfonamid und Natriumhydrid als Base
(6) 6-[N-(2-Dimethylamino-ethyl)-N-methylsulfonyl-amino]-1,3-dinitrobenzol
   Hergestellt aus 2,4-Dinitro-chlorbenzol, N-(2-Dimethylaminoethyl)-methansulfonamid und Natriumhydrid als Base
(7) 4-[N-(2-Dimethylamino-ethyl)-N-methylsulfonyl-amino]-3-chlor-nitrobenzol
   Hergestellt aus 2-Fluor-B-nitro-chlorbenzol, N-(2-Dimethylamino-ethyl)-methansulfonamid und Natriumhydrid als Base
(8) 4-(2-Dimethylamino-ethyl-amino)-1,3-dinitrobenzol
   Hergestellt aus 1-Chlor-2,4-dinitro-benzol und N,N-Dimethylethylendiamin
(9) 4-[N-(2-Dimethylamino-ethyl)-N-(ethylsulfonyl)-amino]-nitrobenzol
   Hergestellt aus 1-Fluor-4-nitro-benzol, N-(2-Dimethylamino-ethyl)-ethansulfonamid und Natriumhydrid als Base
(10) 4-[N-(2-Dimethylamino-ethyl)-N-(propylsulfonyl)-amino]-nitrobenzol
   Hergestellt aus 1-Fluor-4-nitro-benzol, N-(2-Dimethylaminoethyl)-propansulfonamid und Natriumhydrid als Base
(11) 4-[N-(2-Dimethylamino-ethyl)-N-(butylsulfonyl)-amino]-nitrobenzol
   Hergestellt aus 1-Fluor-4-nitro-benzol, N-(2-Dimethylaminoethyl)-butansulfonamid und Natriumhydrid als Base
(12) 4-[N-(2-Dimethylamino-ethyl)-N-(benzylsulfonyl)-amino]-nitrobenzol
   Hergestellt aus 1-Fluor-4-nitro-benzol, N-(2-Dimethylaminoethyl)-C-phenylmethansulfonamid und Natriumhydrid als Base
(13) 4-[N-(2-Dimethylamino-ethyl)-N-(phenylsulfonyl)-amino]-nitrobenzol
   Hergestellt aus 1-Fluor-4-nitro-benzol, N-(2-Dimethylaminoethyl)-benzolsulfonamid und Natriumhydrid als Base
(14) 4-[N-(2-Dimethylamino-ethyl)-N-(isopropylsulfonyl)-amino]-nitrobenzol
   Hergestellt aus 1-Fluor-4-nitro-benzol, N-(2-Dimethylaminoethyl)-isopropylsulfonamid und Natriumhydrid als Base
(15) 4-[N-(2-Dimethylamino-ethyl)-amino]-3-brom-nitrobenzol Hergestellt aus 2-Brom-1-fluor-4-nitro-benzol und N,N-Dimethyl-ethylendiamin
(16) 4-Isopropylamino-nitrobenzol
   Hergestellt aus 1-Fluor-4-nitrobenzol und Isopropylamin
(17) 4-Benzylamino-nitrobenzol
   Hergestellt aus 1-Fluor-4-nitrobenzol und Benzylamin

### Beispiel x

### 4-(Imidazol-4-yl)-nitrobenzol

9.5 g 2-Phenylimidazol werden in 50 ml konzentrierter Schwefelsäure vorsichtig gelöst und zu dieser Lösung werden bei 0°C 5.8 g Ammoniumnitrat zugegeben. Nach weiteren 60 Minuten Rühren bei 0°C wird der Ansatz auf Eiswasser gegossen, mit Ammoniakwasser angebast und der ausgefallene Niederschlag abgesaugt und aus Ethanol umkristallisiert.
Ausbeute: 8.0 g (64 % der Theorie),
R_{f}-Wert: 0.6 (Kieselgel, Essigester/Ethanol = 10:1)
C₉H₇N₃O₂
Massenspektrum: m/z = 189 [M⁻]

Analog Beispiel X werden folgende Verbindungen hergestellt:
(1) 4-(Imidazol-2-yl)-nitrobenzol
   Hergestellt aus 4-(Imidazol-2-yl)-benzol
(2) 4-(5-Methyl-imidazol-4-yl)-nitrobenzol
   Hergestellt aus 4-Methyl-5-phenyl-imidazol (J. Heterocycl. Chem. 1983, 20, 1277-1281)

### Beispiel XI

### 4-(2-(Imidazol-4-yl)-ethylen)-nitrobenzol

1.5 g 4-Nitrobenzaldehyd und 7.45 g (N-Trityl-imidazol-4-yl-methyl)-triphenylphosphoniumchlorid werden in 75 ml Tetrahydrofuran gelöst und zu dieser Lösung werden bei Raumtemperatur 3.0 ml DBU zugetropft. Nach weiteren 120 Minuten Rühren bei Raumtemperatur wird der Ansatz auf Wasser gegossen und der ausgefallene Niederschlag abgesaugt. Das Produkt wird in 25 ml 1N Salzsäure aufgenommen und für 4 Stunden am Rückfluß gekocht. Nach dieser Zeit wird mit Ammoniakwasser neutralisiert, mit Ethylacetat extrahiert und die organische Phase mit Wasser gewaschen, über Natriumsulfat getrocknet und eingedampft. Der Rückstand wird über eine Kieselgel-Säule mit Methylenchlorid/Methanol 10:1 als Laufmittel aufgereinigt.
Ausbeute: 1.0 g (47 % der Theorie),
R_{f}-Wert: 0.6 (Kieselgel, Essigester/Ethanol = 10:1)
Schmelzpunkt: 185-188°C

### Beispiel XII

### 4-(Piperidin-1-yl-methyl)-nitrobenzol

40.0 g 4-Nitrobenzylbromid werden in 500 ml Methylenchlorid gelöst, 51.5 ml Triethylamin zugegeben und 18.3 ml Piperidin vorsichtig zugetropft. Nach Ende der exothermen Reaktion wird für weitere 30 Minuten unter Rückfluß erhitzt. Nach dem Abkühlen wird mit Wasser gewaschen und die organische Phase über Natriumsulfat getrocknet. Schließlich wird die organische Phase eingeengt.
Ausbeute: 36.3 g (89 % der Theorie),
R_{f}-Wert: 0.6 (Kieselgel, Methylenchlorid/Methanol = 9:1) C₁₂H₁₆N₂O₂
Massenspektrum: m/z = 221 [M⁺]

Analog Beispiel XII werden folgende Verbindungen hergestellt:
(1) 4-[(2,6-Dimethyl-piperidin-1-yl)-methyl]-nitrobenzol
(2) 3-(N,N-Dimethyl-aminomethyl)-nitrobenzol
(3) 4-(N,N-Dimethyl-aminomethyl)-nitrobenzol
(4) 4-(2-Dimethylamino-ethyl)-nitrobenzol
(5) 4-(2-Diethylamino-ethyl)-nitrobenzol
(6) 4-(Diethylamino-methyl)-nitrobenzol
(7) 4-(N-Benzyl-N-methyl-aminomethyl)-nitrobenzol
(8) 4-(N-Ethyl-N-methyl-aminomethyl)-nitrobenzol
(9) 4-[N-(n-Hexyl)-N-methyl-aminomethyl]-nitrobenzol
(10) 4-(Thiomorpholin-4-yl-methyl)-nitrobenzol
(11) 4-[(4-Methyl-piperazin-1-yl)-methyl]-nitrobenzol
(12) 4-(Imidazol-1-yl-methyl)-nitrobenzol
(13) 4-[2-(4-Hydroxy-piperidin-1-yl)-ethyl-amino]-nitrobenzol
(14) 4-[(3-Hydroxy-pyrrolidin-1-yl)-methyl]-nitrobenzol
(15) 4-(1,2,4-Triazol-1-yl-methyl)-nitrobenzol
(16) 4-(1,2,3-Triazol-2-yl-methyl)-nitrobenzol
(17) 4-(1,2,3-Triazol-1-yl-methyl)-nitrobenzol
(18) 4-[(N-Ethoxycarbonylmethyl-N-methyl-amino)-methyl]-nitrobenzol
(19) 4-[(N-Aminocarbonylmethyl-N-methyl-amino)-methyl]-nitrobenzol
(20) 4-(Azetidin-1-yl-methyl)-nitrobenzol
(21) 4-[(Di-(2-methoxy-ethyl)-amino)-methyl]-nitrobenzol
(22) 4-[N-(N-tert.Butoxycarbonyl-3-amino-propyl)-N-methylaminomethyl]-nitrobenzol
(23) 4-[(N-Propyl-N-methyl-amino)-methyl]-nitrobenzol
(24) 4-[(N-(2-Dimethylamino-ethyl)-N-methyl-amino)-methyl]-nitrobenzol
(25) 4-[(N-(3-Dimethylamino-propyl)-N-methyl-amino)-methyl]-nitrobenzol
(26) 4-[(N-(2-Methoxy-ethyl)-N-methyl-amino)-methyl]-nitrobenzol
(27) 4-[(N-(2-Hydroxy-ethyl)-N-methyl-amino)-methyl] - nitrobenzol
(28) 4-[(N-(Dioxolan-2-yl-methyl)-N-methyl-amino)-methyl]-nitrobenzol
(29) 4-(3-Oxo-piperazin-1-yl-methyl)-nitrobenzol

### Beispiel XIII

### 4-[(N-carboxymethyl-N-methyl-amino)-methyl]-nitrobenzol

7.33 g 4-[(N-Ethoxycarbonylmethyl-N-methyl-amino)-methyl]-nitrobenzol werden in 140 ml Ethanol gelöst, 34.0 ml 1N Natronlauge zugegeben und das Gemisch eine halbe Stunde bei Raumtemperatur gerührt. Nach dieser Zeit wird mit 34 ml 1N Salzsäure neutralisiert, das Lösungsmittel abgezogen, der Rückstand in Methylenchlorid aufgenommen und mit Wasser extrahiert. Die wäßrige Phase wird eingeengt und der Rückstand aus Methylenchlorid umkristallisiert.
Ausbeute: 5.43 g (84 % der Theorie),
R_{f}-Wert: 0.4 (Kieselgel, Methylenchlorid/Methanol = 2:1) C₁₀H₁₂N₂O₄
Massenspektrum: m/z = 223 [M⁺]

### Beispiel XIV

### 4-(N-Ethyl-aminomethyl)-nitrobenzol

6.0 g 4-Nitrobenzylbromid werden in 25 ml Ethanol gelöst, mit 25 ml 10%iger ethanolischer Ethylaminlösung versetzt und 2 Stunden am Rückfluß gekocht. Dann wird die Lösung einrotiert, der Rückstand mit Methylenchlorid aufgenommen und mit verdünnter Natronlauge gewaschen. Schließlich wird die organische Phase eingeengt.
Ausbeute: 2.3 g (46 % der Theorie),
R_{f}-Wert: 0.2 (Kieselgel, Methylenchlorid/Methanol = 9:1) C₉H₁₂N₂O₂
ESI-Massenspektrum: m/z = 179 [M-H⁻]

Analog Beispiel XIV werden folgende Verbindungen hergestellt:
(1) 4-[N-(4-Chlorbenzyl)-aminomethyl]-nitrobenzol
(2) 4-(N-Cyclohexyl-aminomethyl)-nitrobenzol
(3) 4-(N-Isopropyl-aminomethyl)-nitrobenzol
(4) 4-(N-Propyl-aminomethyl)-nitrobenzol
(5) 4-(N-Methyl-aminomethyl)-nitrobenzol
(6) 4-(N-Butyl-aminomethyl)-nitrobenzol
(7) 4-(N-Methoxycarbonylmethyl-aminomethyl)-nitrobenzol
(8) 4-(N-Benzyl-aminomethyl)-nitrobenzol
(9) 4-(Aminomethyl)-nitrobenzol
(10) 4-(Pyrrolidin-1-yl-methyl)-nitrobenzol
(11) 4-(Morpholin-4-yl-methyl)-nitrobenzol
(12) 4-(Hexamethyleniminomethyl)-nitrobenzol
(13) 4-(4-Hydroxy-piperidin-1-yl-methyl)-nitrobenzol
(14) 4-(4-Methoxy-piperidin-1-yl-methyl)-nitrobenzol
(15) 4-(4-Methyl-piperidin-1-yl-methyl)-nitrobenzol
(16) 4-(4-Ethyl-piperidin-1-yl-methyl)-nitrobenzol
(17) 4-(4-Isopropyl-piperidin-1-yl-methyl)-nitrobenzol
(18) 4-(4-Phenyl-piperidin-1-yl-methyl)-nitrobenzol
(19) 4-(4-Benzyl-piperidin-1-yl-methyl)-nitrobenzol
(20) 4-(4-Ethoxycarbonyl-piperidin-1-yl-methyl)-nitrobenzol
(21) 4-(N,N-Dipropyl-aminomethyl)-nitrobenzol
(22) 4-(4-tert.Butoxycarbonyl-piperazin-1-yl-methyl)-nitrobenzol
(23) 4-(2-Morpholin-4-yl-ethyl)-nitrobenzol
(24) 4-(2-Pyrrolidin-1-yl-ethyl)-nitrobenzol
(25) 4-(2-Piperidin-1-yl-ethyl)-nitrobenzol
(26) 4-(N-Ethyl-N-benzyl-aminomethyl)-nitrobenzol
(27) 4-(N-Propyl-N-benzyl-aminomethyl)-nitrobenzol
(28) 4-[N-Methyl-N-(4-chlorbenzyl)-aminomethyl]-nitrobenzol
(29) 4-[N-Methyl-N-(4-brombenzyl)-aminomethyl]-nitrobenzol
(30) 4-[N-Methyl-N-(4-fluorbenzyl)-aminomethyl]-nitrobenzol
(31) 4-[N-Methyl-N-(4-methylbenzyl)-aminomethyl]-nitrobenzol
(32) 4-[N-Methyl-N-(3-chlorbenzyl)-aminomethyl]-nitrobenzol
(33) 4-[N-Methyl-N-(3,4-dimethoxybenzyl)-aminomethyl]-nitrobenzol
(34) 4-[N-Methyl-N-(4-methoxybenzyl)-aminomethyl]-nitrobenzol
(35) 4-(N-2,2,2-Trifluorethyl-N-benzyl-aminomethyl)-nitrobenzol
(36) 4-[N-2,2,2-Trifluorethyl-N-(4-chlorbenzyl)-aminomethyl]-nitrobenzol
(37) 4-(Thiomorpholin-4-yl-methyl)-nitrobenzol
(38) 4-(Azetidion-1-yl-rnethyl)-nitrobenzol
(39) 4-(3,4-Dihydropyrrolidin-1-yl-methyl)-nitrobenzol
(40) 4-(3,4-Dihydropiperidin-1-yl-methyl)-nitrobenzol
(41) 4-(2-Methoxycarbonyl-pyrrolidin-1-yl-methyl)-nitrobenzol
(42) 4-(3,5-Dimethyl-piperidin-1-yl-methyl)-nitrobenzol
(43) 4-(4-Phenyl-piperazin-1-yl-methyl)-nitrobenzol
(44) 4-(4-Phenyl-4-hydroxy-piperidin-1-yl-methyl)-nitrobenzol
(45) 4-[N-(3,4,5-Trimethoxybenzyl-N-methyl-aminomethyl)-nitrobenzol
(46) 4-[N-(3,4-Dimethoxybenzyl)-N-ethyl-aminomethyl]-nitrobenzol
(47) 4-[N-(2,6-Dichlorbenzyl)-N-methyl)-aminomethyl]-nitrobenzol
(48) 4-[N-(4-Trifluormethylbenzyl)-N-methyl)-aminomethyl]-nitrobenzol
(49) 4-(N-Benzyl-N-isopropyl-aminomethyl)-nitrobenzol
(50) 4-(N-Benzyl-N-tert.butyl-aminomethyl)-nitrobenzol
(51) 4-(N,N-Diisopropyl-aminomethyl)-nitrobenzol
(52) 4-(N,N-Diisobutyl-aminomethyl)-nitrobenzol
(53) 4-(2,3,4,5-Tetrahydro-benzo(d)azepin-3-yl-methyl)-nitrobenzol
(54) 4-(2,3-Dihydro-isoindol-2-yl-methyl)-nitrobenzol
(55) 4-(6,7-Dimethoxy-1,2,3,4-tetrahydro-isoquinolin-2-yl-methyl)-nitrobenzol
(56) 4-(1,2,3,4-Tetrahydro-isoquinolin-2-yl-methyl)-nitrobenzol
(57) 4-[N-(2-Hydroxyethyl)-N-benzyl-aminomethyl]-nitrobenzol
(58) 4-[N-(1-Ethyl-pentyl)-N-(pyridin-2-yl-methyl)-aminomethyll-nitrobenzol
(59) 4-(Piperin-1-yl-methyl)-1,3-dinitrobenzol
(60) 4-(N-Phenethyl-N-methyl-aminomethyl)-nitrobenzol
(61) 4-[N-(3,4-Dihydroxy-phenethyl)-N-methyl-aminomethyl]-nitrobenzol
(62) 4-[N-(3,4,5-Trimethoxy-phenethyl)-N-methyl-aminomethyl]-nitrobenzol
(63) 4-[N-(3,4-Dimethoxy-phenethyl)-N-methyl-aminomethyl]-nitrobenzol
(64) 4-[N-(3,4-Dimethoxy-benzyl)-N-methyl-aminomethyl]-nitrobenzol
(65) 4-[N-(4-Chlor-benzyl)-N-methyl-aminomethyl]-nitrobenzol
(66) 4-[N-(4-Brom-benzyl)-N-methyl-aminomethyl]-nitrobenzol
(67) 4-[N-(4-Fluor-benzyl)-N-methyl-aminomethyl]-nitrobenzol
(68) 4-[N-(4-Methyl-benzyl)-N-methyl-aminomethyl]-nitrobenzol
(69) 4-[N-(4-Nitro-phenethyl)-N-methyl-aminomethyl]-nitrobenzol
(70) 4-(N-Phenethyl-N-benzyl-aminomethyl)-nitrobenzol
(71) 4-(N-Phenethyl-N-cyclohexyl-aminomethyl)-nitrobenzol
(72) 4-[N-(2-(Pyridin-2-yl)-ethyl)-N-methyl-aminomethyl]-nitrobenzol
(73) 4-[N-(2-(Pyridin-4-yl)-ethyl)-N-methyl-aminomethyl]-nitrobenzol
(74) 4-[N-(Pyridin-4-yl-methyl)-N-methyl-aminomethyl]-nitrobenzol
(75) 4-(N,N-Dibenzyl-aminomethyl)-nitrobenzol
(76) 4-[N-(4-Nitro-phenethyl)-N-propyl-aminomethyl]-nitrobenzol
(77) 4-(N-Benzyl-N-(3-cyano-propyl)-aminomethyl)-nitrobenzol
(78) 4-(N-Benzyl-N-allyl-aminomethyl)-nitrobenzol
(79) 4-[N-Benzyl-N-(2,2,2-trifluorethyl)-aminomethyl]-nitrobenzol
(80) 4-[N-(2-Benzo(1,3)dioxol-5-yl-methyl)-N-methylaminomethyl]-nitrobenzol
(81) 4-(7-Chlor-2,3.,4,5-tetrahydro-benzo(d)azepin-3-yl-methyl)-nitrobenzol
(82) 4-(7,8-Dichlor-2,3,4,5-tetrahydro-benzo(d)azepin-3-yl-methyl)-nitrobenzol
(83) 4-(7-Methoxy-2,3,4,5-tetrahydro-benzo(d)azepin-3-yl-methyl)-nitrobenzol
(84) 4-(7-Methyl-2,3.,4,5-tetrahydro-benzo(d)azepin-3-yl-methyl)-nitrobenzol
(85) 4-(7,8-Dimethoxy-2,3,4,5-tetrahydro-benzo(d)azepin-3-yl-methyl)-nitrobenzol
(86) 4-(6,7-Dichlor-1,2,3,4-tetrahydro-isoquinolin-2-yl-methyl)-nitrobenzol
(87) 4-(6,7-Dimethyl-1,2,3,4-tetrahydro-isoquinolin-2-yl-methyl)-nitrobenzol
(88) 4-(6-Chlor-1,2,3,4-tetrahydro-isoquinolin-2-yl-methyl)-nitrobenzol
(89) 4-(7-Chlor-1,2,3,4-tetrahydro-isoquinolin-2-yl-methyl)-nitrobenzol
(90) 4-(6-Methoxy-1,2,3,4-tetrahydro-isoquinolin-2-yl-methyl)-nitrobenzol
(91) 4-(7-Methoxy-1,2,3,4-tetrahydro-isoquinolin-2-yl-methyl)-nitrobenzol
(92) 4-[(2,3,4,5-Tetrahydro-azepino(4,5-b)pyrazin-3-yl)-methyl]-nitrobenzol
(93) 4-[(7-Amino-2,3,4,5-tetrahydro-azepino(4,5-b)pyrazin-3-yl)-methyl]-nitrobenzol
(94) 4-[(2-Amino-5,6,7,8-tetrahydro-azepino(4,5-d)thiazol-6-yl)-methyl]-nitrobenzol
(95) 4-[(5,6,7,8-Tetrahydro-azepino(4,5-d)thiazol-6-yl)-methyl]-nitrobenzol

### Beispiel XV

### 4-(1,1-Dioxo-thiomorpholin-4-yl-methyl)-nitrobenzol

6.0 g 4-(Thiomorpholin-4-yl-methyl)-nitrobenzol werden in 100 ml Methylenchlorid gelöst und 10.3 g meta-Chlorperbenzoesäure langsam zugegeben. Nach weiteren 3 Stunden Rühren bei Raumtemperatur wird der erhaltene Niederschlag abfiltriert.
Ausbeute: 6.2 g (91 % der Theorie)
R_{f}-Wert: 0.5 (Kieselgel, Methylenchlorid/Methanol = 1:1) C₁₁H₁₄N₂O₄S
Massenspektrum: m/z = 270 [M⁺]

Analog Beispiel XV wird folgende Verbindung hergestellt:

### (1) 4-(1-Oxo-thiomorpholin-4-yl-methyl)-nitrobenzol

### Beispiel XVI

### 4-[N-(3-Amino-propyl)-N-methylsulfonyl-amino]-nitrobenzol

9.5 g 4-LN-(3-Phthalimido-2-yl-propyl)-N-methylsulfonyl-amino]-nitrobenzol werden in 200 ml Ethanol gelöst, 11.5 ml Hydrazinhydrat zugegeben und das Gemisch 1.5 Stunden bei 50 °C gerührt. Nach dem Abkühlen wird der Rückstand weitgehend eingeengt, Wasser zugegeben und die Lösung mit Methylenchlorid extrahiert. Die organische Phase wird getrocknet, eingeengt und über eine Kieselgelsäule mit Methylenchlorid/Methanol/Ammoniak 9:1:0.1 aufgereinigt.
Ausbeute: 2.5 g (39 % der Theorie)
R_{f}-Wert: 0.2 (Kieselgel, Methylenchlorid/Methanol = 9:1) C₁₀H₁₅N₃O₄S
ESI-Massenspektrum: m/z = 272 [M-H⁻]

Analog Beispiel XVI wird folgende Verbindung hergestellt:
(1) 6-[N-(2-Dimethylamino-ethyl)-N-methylsulfonyl-amino]-3-amino-nitrobenzol
   Hergestellt aus 6-[N-(2-Dimethylamino-ethyl)-N-methylsulfonyl-amino]-3-phthalimido-2-yl-nitrobenzol

### Beispiel XVII

### 4-(1-Methyl-imidazol-2-yl)-nitrobenzol

7.5 g 4-(Imidazol-2-yl)-nitrobenzol werden in 50 ml Dimethylsulfoxid gelöst und bei 0°C 5.0 g Kalium-tert.butylat zugegeben. Nach einer Stunde Rühren bei Raumtemperatur werden 2.6 ml Methyliodid zugetropft und das Gemisch eine Stunde bei Raumtemperatur gerührt. Nach dieser Zeit wird der Rückstand auf Eiswasser gegossen und der entstandene Niederschlag abgesaugt, mit Wasser gewschen und getrocknet.
Ausbeute: 6.1 g (76 % der Theorie)
R_{f}-Wert: 0.6 (Kieselgel, Methylenchlorid/Methanol = 10:1) Schmelzpunkt: 186-187°C

Analog Beispiel XVII werden folgende Verbindungen hergestellt:
(1) 4-(1-Ethyl-imidazol-2-yl)-nitrobenzol
   Hergestellt aus 4-(Imidazol-2-yl)-nitrobenzol und Ethyliodid
(2) 4-(1-Benzyl-imidazol-2-yl)-nitrobenzol
   Hergestellt aus 4-(Imidazol-2-yl)-nitrobenzol und Benzylbromid

### Beispiel XVIII

### 4-[(N-(2-(2-Methoxy-ethoxy)-ethyl)-N-methyl-amino)-methyl]-nitrobenzol

5.0 g 4-Methylaminomethyl-nitrobenzol werden in 30 ml Dimethylformamid gelöst und 4.6 g 2-(2-Methoxy-ethoxy)-ethylchlorid zugegeben. Nach sechs Stunden Rühren bei 100°C wird das Lösungsmittel abgezogen und der Rückstand in Essigester aufgenommen. Die organische Phase wird mit Wasser gewaschen und über Natriumsulfat getrocknet. Nach Abziehen des Lösungsmittels wird der Rückstand über eine AluminiumoxidSäule (Aktivität 2-3) mit Toluol/Ethylacetat 5:1 als Laufmittel aufgereinigt.
Ausbeute: 2.3 g (29 % der Theorie)
R_{f}-Wert: 0.5 (Aluminiumoxid, Toluol/Ethylacetat = 5:1)
C₁₃H₂₀N₂O₄
ESI-Massenspektrum: m/z = 267 [M-H⁻]

### Beispiel XIX

### 4-(N-Ethyl-N-tert. butoxycarbonyl-aminomethyl)-nitrobenzol

2.2 g 4-(Ethylaminomethyl)-nitrobenzol werden in 50 ml Essigester gelöst und mit 2.6 g Di-tert-butyl.dicarbonat (tert.Butoxycarbonyl-anhydrid) 30 Minuten bei Raumtemperatur gerührt. Anschließend wird die Lösung mit Wasser gewaschen und eingeengt.
Ausbeute: 3.4 g der Theorie
R_{f}-Wert: 0.3 (Kieselgel, Methylenchlorid/Methanol = 50:1) Schmelzpunkt: 85 °C

Analog Beispiel XIX werden folgende Verbindungen hergestellt:
(1) 4-[N-(4-Chlorphenyl-methyl)-N-tert.butoxycarbonyl-aminomethyl]-nitrobenzol
(2) 4-(N-tert.Butoxycarbonyl-aminomethyl)-nitrobenzol
(3) 4-(N-Cyclohexyl-N-tert.butoxycarbonyl-aminomethyl)-nitrobenzol
(4) 4-(N-Isopropyl-N-tert.butoxycarbonyl-aminomethyl)-nitrobenzol
(5) 4-(N-Methyl-N-tert.butoxycarbonyl-aminomethyl)-nitrobenzol
(6) 4-(N-Propyl-N-tert.butoxycarbonyl-aminomethyl)-nitrobenzol
(7) 4-(N-Butyl-N-tert.butoxycarbonyl-aminomethyl)-nitrobenzol
(8) 4-(N-Methoxycarbonylmethyl-N-tert.butoxycarbonyl-aminomethyl)-nitrobenzol
(9) 4-(N-Benzyl-N-tert.butoxycarbonyl-aminomethyl)-nitrobenzol
(10) 4-[N-(3-Trifluoracetylamino-propyl)-N-methylsulfonyl-amino]-nitrobenzol
   Hergestellt aus 4-[N-(3-Amino-propyl)-N-methylsulfonyl-amino]-nitrobenzol und Trifluoressigsäureanhydrid
(11) 4-[(4-tert.Butoxycarbonyl-piperazin-1-yl)-methyl]-nitrobenzol

### Beispiel XX

### 4-(Piperidin-1-yl-methyl)-anilin

37.0 g 4-(Piperidin-1-yl-methyl)-nitrobenzol werden in 300 ml Methanol gelöst, 8.0 g Raney-Nickel zugegeben und für 85 Minuten mit 3 bar Wasserstoff bei Raumtemperatur hydriert. Der Katalysator wird abfiltriert und das Filtrat eingedampft.
Ausbeute: 24.0 g (75 % der Theorie),
R_{f}-Wert: 0.4 (Kieselgel, Methylenchlorid/Methanol = 9:1)
C₁₂H₁₈N₂
ESI-Massenspektrum: m/z = 191 [M+H⁺]

Analog Beispiel VIII werden folgende Verbindungen hergestellt:
(1) 4-[(2,6-Dimethyl-piperidin-1-yl)-methyl]-anilin
(2) N-(2-Dimethylamino-ethyl)-N-methylsulfonyl-p-phenylendiamin
(3) 3-(Dimethylaminomethyl)-anilin
(4) 4-(Dimethylaminomethyl)-anilin
(5) 4-(2-Dimethylamino-ethyl)-anilin
(6) 4-[N-(2-Dimethylamino-ethyl)-N-acetyl-amino]-anilin
(7) 4-[N-(3-Dimethylamino-propyl)-N-acetyl-amino]-anilin
(8) 4-[N-(2-Dimethylamino-ethyl)-N-benzoyl-amino]-anilin
(9) 4-[N-(2-Dimethylamino-ethyl)-N-propionyl-amino]-anilin
(10) 4-[N-(2-Dimethylamino-ethyl)-N-butyryl-amino]-anilin
(11) 4-[N-(2-Dimethylamino-ethyl)-N-isobutyryl-amino]-anilin
(12) 4-(N-tert.Butoxycarbonyl-aminomethyl)-anilin
(13) 4-(N-Ethyl-N-tert.butoxycarbonyl-aminomethyl)-anilin
(14) 4-[N-(4-Chlorphenyl-methyl)-N-tert.butoxycarbonylaminomethyl]-anilin
(15) 4-(N-Cyclohexyl-N-tert.butoxycarbonyl-aminomethyl)-anilin
(16) 4-(N-Isopropyl-N-tert.butoxycarbonyl-aminomethyl)-anilin
(17) 4-(N-Propyl-N-tert.butoxycarbonyl-aminomethyl)-anilin
(18) 4-(N-Methyl-N-tert.butoxycarbonyl-aminomethyl)-anilin
(19) 4-(N-Butyl-N-tert.butoxycarbonyl-aminomethyl)-anilin
(20) 4-(N-Methoxycarbonyl-methyl-N-tert.butoxycarbonylaminomethyl)-anilin
(21) 4-(N-Benzyl-N-tert.butoxycarbonyl-aminornethyl)-anilin
(22) 4-(Pyrrolidin-1-yl-methyl)-anilin
(23) 4-(Morpholin-4-yl-methyl)-anilin
(24) 4-(Hexamethyleniminomethyl)-anilin
(25) 4-(4-Hydroxy-piperidin-1-yl-methyl)-anilin
(26) 4-(4-Methoxy-piperidin-1-yl-methyl)-anilin
(27) 4-(4-Methyl-piperidin-1-yl-methyl)-anilin
(28) 4-(4 -Ethyl-piperidin-1-yl-methyl)-anilin
(29) 4-(4-Isopropyl-piperidin-1-yl-methyl)-anilin
(30) 4-(4-Phenyl-piperidin-1-yl-methyl)-anilin
(31) 4-(4-Benzyl-piperidin-1-yl-methyl)-anilin
(32) 4-(4-Ethoxycarbonyl-piperidin-1-yl-methyl)-anilin
(33) 4-(N,N-Dipropyl-aminomethyl)-anilin
(34) 4-(4-tert.Butoxycarbonyl-piperazin-1-yl-methyl)-anilin
(35) 4-(2-Morpholin-4-yl-ethyl)-anilin
(36) 4-(2-Pyrrolidin-1-yl-ethyl)-anilin
(37) 4-(2-Piperidin-1-yl-ethyl)-anilin
(38) 4-(N-Propyl-N-benzyl-aminomethyl)-anilin
(39) 4-[N-(n-Hexyl)-N-methyl-aminomethyl]-anilin
(40) 4-[N-Methyl-N-(4-chlorbenzyl)-aminomethyl]-anilin
(41) 4-[N-Methyl-N-(4-brombenzyl)-aminomethyl]-anilin
(42) 4-[N-Methyl-N-(4-methylbenzyl)-aminomethyl]-anilin
(43) 4-[N-Methyl-N-(4-fluorbenzyl)-aminomethyl]-anilin
(44) 4-[N-Methyl-N-(3-chlorbenzyl)-aminomethyl]-anilin
(45) 4-[N-Methyl-N-(3,4-dimethoxybenzyl)-aminomethyl]-anilin
(46) 4-[N-Methyl-N-(4-methoxybenzyl)-aminomethyl]-anilin
(47) 4-(N-2,2,2-Trifluoroethyl-N-benzyl-aminomethyl)-anilin
(48) 4-[N-2,2,2-Trifluorethyl-N-(4-chlorbenzyl)-aminomethyl]-anilin
(49) 4-(Thiomorpholin-4-yl-methyl)-anilin
(50) 4-(1-Oxo-thiomorpholin-4-yl-methyl)-anilin
(51) 4-(1,1-Dioxo-thiomorpholin-4-yl-methyl)-anilin
(52) 4-(Azetidion-1-yl-methyl)-anilin
(53) 4-(3,4-Dihydropyrrolidin-1-yl-methyl)-anilin
(54) 4-(3,4-Dihydropiperidin-1-yl-methyl)-anilin
(55) 4-(2-Methoxycarbonyl-pyrrolidin-1-yl-methyl)-anilin
(56) 4-(3,5-Dimethyl-piperidin-1-yl-methyl)-anilin
(57) 4-(4-Phenyl-piperazin-1-yl-methyl)-anilin
(58) 4-(4-Phenyl-4-hydroxy-piperidin-1-yl-methyl)-anilin
(59) 4-[N-(3,4,5-Trimethoxy-benzyl)-N-methyl-aminomethyl]-anilin
(60) 4-[N-(3,4-Dimethoxy-benzyl)-N-ethyl-aminomethyl]-anilin
(61) 4-(N-Benzyl-N-ethyl-aminomethyl)-anilin
(62) 4-[N-(2,6-Dichlorbenzyl)-N-methyl-aminomethyl]-anilin
(63) 4-[N-(4-Trifluormethylbenzyl)-N-methyl-aminomethyl]-anilin
(64) 4-(N-Benzyl-N-isopropyl-aminomethyl)-anilin
(65) 4-(N-Benzyl-N-tert.butyl-aminomethyl)-anilin
(66) 4-(Diethylamino-methyl)-anilin
(67) 4-(2-Diethylamino-ethyl)-anilin
(68) 4-(N,N-Diisopropyl-aminomethyl)-anilin
(69) 4-(N,N-Diisobutyl-aminomethyl)-anilin
(70) 4-(2,3,4,5-Tetrahydro-benzo(d)azepin-3-yl-methyl)-anilin
(71) 4-(2,3-Dihydro-isoindol-2-yl-methyl)-anilin
(72) 4-(6,7-Dimethoxy-1,2,3,4-tetrahydro-isoquinolin-2-yl-methyl)-anilin
(73) 4-(1,2,3,4-Tetrahydro-isoquinolin-2-yl-methyl)-anilin
(74) 4-[N-(2-Hydroxy-ethyl)-N-benzyl-aminomethyl]-anilin
(75) 4-[N-(1-Ethyl-pentyl)-N-(pyridin-2-yl-methyl)-aminomethyl]-anilin
(76) 4-(Piperidin-1-yl-methyl)-3-nitro-anilin
(77) 4-(Piperidin-1-yl-methyl)-3-amino-anilin
(78) 4-(N-Benzyl-N-methyl-aminomethyl)-anilin
(79) 4-(N-Ethyl-N-methyl-aminomethyl)-anilin
(80) 4-(N-Phenethyl-N-methyl-aminomethyl)-anilin
(81) 4-[N-(3,4-Dihydroxy-phenethyl)-N-methyl-aminomethyl]-anilin
(82) 4-[N-(3,4,5-Trimethoxy-phenethyl)-N-methyl-aminomethyl]-anilin
(83) 4-[N-(3,4-Dimethoxy-phenethyl)-N-methyl-aminomethyl]-anilin
(84) 4-[N-(3,4-Dimethoxy-benzyl)-N-methyl-aminomethyl]-anilin
(85) 4-[N-(4-Chlor-benzyl)-N-methyl-aminomethyl]-anilin
(86) 4-[N-(4-Brom-benzyl)-N-methyl-aminomethyl]-anilin
(87) 4-[N-(4-Fluor-benzyl)-N-methyl-aminomethyl]-anilin
(88) 4-[N-(4-Methyl-benzyl)-N-methyl-aminomethyl]-anilin
(89) 4-[N-(4-Nitro-phenethyl)-N-methyl-arninomethyl]-anilin
(90) 4-(N-Phenethyl-N-benzyl-aminomethyl)-anilin
(91) 4-(N-Phenethyl-N-cyclohexyl-aminomethyl)-anilin
(92) 4-[N-(2-(Pyridin-2-yl)-ethyl)-N-methyl-aminomethyl]-anilin
(93) 4-[N-(2-(Pyridin-4-yl)-ethyl)-N-methyl-aminomethyl]-anilin
(94) 4-[N-(Pyridin-4-yl-methyl)-N-methyl-aminomethyl]-anilin
(95) 4-(N,N-Dibenzylaminomethyl)-anilin
(96) 4-[N-(4-Nitro-benzyl)-N-propyl-aminomethyl]-anilin
(97) 4-[N-Benzyl-N-(3-cyano-propyl)-aminomethyl]-anilin
(98) 4-(N-Benzyl-N-allyl-aminomethyl)-anilin
(99) 4-[N-Benzyl-N-(2,2,2-trifluorethyl)-aminomethyl]-anilin
(100) 4-[(Benzo(1,3)dioxol-5-yl-methyl)-methyl-aminomethyl]-anilin
(101) 4-(7-Chlor-2,3,4,5-tetrahydro-benzo(d)azepin-3-yl-methyl)-anilin
(102) 4-(7,8-Dichlor-2,3,4,5-tetrahydro-benzo(d)azepin-3-yl-methyl)-anilin,
(103) 4-(7-Methoxy-2,3,4,5-tetrahydro-benzo(d)azepin-3-yl-methyl)-anilin
(104) 4-(7-Methyl-2,3,4,5-tetrahydro-benzo(d) azepin-3-yl-methyl)-anilin
(105) 4-(7,8-Dimethoxy-2,3,4,5-tetrahydro-benzo(d)azepin-3-yl-methyl)-anilin
(106) 4-(6,7-Dichlor-1,2,3,4-tetrahydro-isoquinolin-2-yl-methyl)-anilin
(107) 4-(6,7-Dimethyl-1,2,3,4-tetrahydro-isoquinolin-2-yl-methyl)-anilin
(108) 4-(6-Chlor-1,2,3,4-tetrahydro-isoquinolin-2-yl-methyl)-anilin
(109) 4-(7-Chlor-1,2,3,4-tetrahydro-isoquinolin-2-yl-methyl)-anilin
(110) 4-(6-Methoxy-1,2,3,4-tetrahydro-isoquinolin-2-yl-methyl)-anilin
(111) 4-(7-Methoxy-1,2,3,4-tetrahydro-isoquinolin-2-yl-methyl)-anilin
(112) 4-(2,3,4,5-Tetrahydro-azepino(4,5-b)pyrazin-3-yl-methyl)-anilin
(113) 4-(7-Amino-2,3,4,5-tetrahydro-azepino(4,5-b)pyrazin-3-yl-methyl)-anilin
(114) 4-(2-Amino-5,6,7,8-tetrahydro-azepino(4,5-d)thiazol-6-yl-methyl)-anilin
(115) 4-(5,6,7,8-Tetrahydro-azepino(4,5-d)thiazol-6-yl-methyl)-anilin
(116) 4-(4-Methyl-piperazin-1-yl)-anilin
(117) 4-[N-(2-Dimethylamino-ethyl)-N-methyl-amino]-anilin
(118) 4-[N-(3-Dimethylamino-propyl)-N-methyl-amino]-anilin
(119) N-(3-Dimethylamino-propyl)-N-methylsulfonyl-p-phenylendiamin
(120) 4-[(N-Dimethylaminocarbonylmethyl-N-methylsulfonyl)-amino] -anilin
(121) N-(4-Aminophenyl)-N-methyl-methansulfonamid
(122) 4-(Imidazol-4-yl)-anilin
(123) 4-(Tetrazol-5-yl)-anilin
(124) 4-[N-(2-Dimethylamino-ethyl)-N-propionyl-amino]-anilin
(125) N-(Dimethylaminomethylcarbonyl)-N-methyl-p-phenylendiamin
(126) N-[(2-Dimethylamino-ethyl)-carbonyl]-N-methyl-p-phenylendiamin
(127) 4-(N-Acetyl-N-dimethylaminocarbonylmethyl)-amino)-anilin
(128) N-Methylaminocarbonylmethyl-N-methylsulfonyl-p-phenylendiamin
(129) N-Aminocarbonylmethyl-N-methylsulfonyl-p-phenylendiamin
(130) 4-(Imidazolidin-2,4-dion-5-yliden-methyl)-anilin
(131) 4-(Imidazolidin-2,4-dion-5-yl-methyl)-anilin
(132) 4-(2-Oxo-pyrrolidin-l-yl-methyl)-anilin
(133) N-Cyanomethyl-N-methylsulfonyl-p-phenylendiamin
(134) 4-[2-(Imidazol-4-yl)-ethyl]-anilin
(135) 4-[(4-Methyl-piperazin-1-yl)-methyl]-anilin
(136) 4-[N-(2-(N-Benzyl-N-methyl-amino)-ethyl)-N-methylsulfonyl-amino]-anilin
(137) 4-[N-(3-(N-Benzyl-N-methyl-amino)-propyl)-N-methylsulfonyl-amino]-anilin
(138) N-Cyclohexyl-p-phenylendiamin
(139) 4-(Pyridin-4-yl-methyl)-anilin
(140) 4-(Imidazol-1-yl-methyl)-anilin
(141) 4-Benzyl-anilin
(142) N-(3-Trifluoracetylamino-propyl)-N-methylsulfonyl-p-phenylendiamin
(143) 4-Amino-phenylessigsäure-tert.butylester
(144) 4-(Imidazol-2-yl)-anilin
(145) 4-(1-Methyl-imidazol-2-yl)-anilin
(146) 4-(1-Ethyl-imidazol-2-yl)-anilin
(147) 4-(1-Benzyl-imidazol-2-yl)-anilin
(148) 4-[N-(2-Dimethylamino-ethyl)-N-methylsulfonyl-amino]-3-amino-anilin
(149) 4-[N-(2-Dimethylamino-ethyl)-N-methylsulfonyl-amino]-3-chlor-anilin
(150) 4-[N-(2-Dimethylamino-ethyl)-N-acetyl-amino]-3-amino-anilin
(151) 4-[N-(2-Dimethylamino-ethyl)-N-acetyl-amino]-3-bromanilin
(152) 4-[2-(4-Hydroxy-piperidin-1-yl)-ethyl-amino]-anilin
(153) N-(2-Dimethylamino-ethyl)-N-ethylsulfonyl-p-phenylendiamin
(154) N-(2-Dimethylamino-ethyl)-N-propylsulfonyl-p-phenylendiamin
(155) N-(2-Dimethylamino-ethyl)-N-isopropylsulfonyl-p-phenylendiamin
(156) N-(2-Dimethylamino-ethyl)-N-butylsulfonyl-p-phenylendiamin
(157) N-(2-Dimethylamino-ethyl)-N-benzylsulfonyl-p-phenylendiamin
(158) N-(2-Dimethylamino-ethyl)-N-phenylsulfonyl-p-phenylendiamin
(159) 4-((3-Hydroxy-pyrrolidin-1-yl)-methyl)-anilin
(160) 4-[N-(2-Dimethylamino-ethyl)-N-(furan-2-carbonyl)-amino]-anilin
(161) 4-[N-(2-Dimethylamino-ethyl)-N-(2-methoxy-benzoyl)-amino]-anilin
(162) 4-[N-(2-Dimethylamino-ethyl)-N-(pyridin-3-carbonyl)-amino]-anilin
(163) 4-[N-(2-Dimethylamino-ethyl)-N-(phenyl-acetyl)-amino]-anilin
(164) N-(Piperidin-1-yl-methylcarbonyl)-N-methyl-p-phenylendiamin
(165) N-(Morpholin-4-yl-methylcarbonyl)-N-methyl-p-phenylendiamin
(166) N-[(4-Benzyl-piperazin-1-yl)-methylcarbonyl]-N-methyl-p-phenylendiamin
(167) N-(Pyrrolidin-1-yl-methylcarbonyl)-N-methyl-p-phenylendiamin
(168) 4-(5-Methyl-imidazol-4-yl)-anilin
(169) N-[(2-Dimethylamino-ethyl)-carbonyl]-N-isopropyl-p-phenylendiamin
(170) N-[(2-Dimethylamino-ethyl)-carbonyl]-N-benzyl-p-phenylendiamin
(171) N-(N-Aminocarbonylmethyl-N-methyl-amino)-methylcarbonyl)-N-methyl-p-phenylendiamin
(172) N-[(N-Benzyl-N-methyl-amino)-methylcarbonyl]-N-methyl-p-phenylendiamin
(173) N-[Di-(2-methoxyethyl)-amino-methylcarbonyl]-N-methyl-p-phenylendiamin
(174) N-[(2-(4-tert.Butoxycarbonyl-piperazin-1-yl)-ethyl)-carbonyl]-N-methyl-p-phenylendiamin
(175) N-[(2-(Piperidin-1-yl)-ethyl)-carbonyl]-N-methyl-p-phenylendiamin
(176) N-[(2-(N-Ben2y1-N-methyl-amino)-ethyl)-carbonyl]-N-methyl-p-phenylendiamin
(177) N-(Dimethylaminomethylcarbonyl)-N-isopropyl-p-phenylendiamin
(178) N-(Piperidin-1-yl-methylcarbonyl)-N-isopropyl-p-phenylendiamin
(179) N-[(4-tert.Butoxycarbonyl-piperazin-1-yl)-methylcarbonyl]-N-isopropyl-p-phenylendiamin
(180) N-[(N-Benzyl-N-methyl-amino)-methylcarbonyl]-N-benzyl-p-phenylendiamin
(181) N-(Dimethylaminomethylcarbonyl)-N-benzyl-p-phenylendiamin
(182) N-(Piperidin-1-yl-methylcarbonyl)-N-benzyl-p-phenylendiamin
(183) 4-(1,2,4-Triazol-1-yl-methyl)-anilin
(184) 4-(1,2,3-Triazol-2-yl-methyl)-anilin
(185) 4-(1,2,3-Triazol-1-yl-methyl)-anilin
(186) 4-[(N-Ethoxycarbonylmethyl-N-methyl-amino)-methyl]-anilin
(187) 4-[(N-Aminocarbonylmethyl-N-methyl-amino)-methyl]-anilin
(188) 4-(Azetidin-1-yl-methyl)-anilin
(189) 4-[(Di-(2-methoxy-ethyl)-amino)-methyl]-anilin
(190) 4-[(N-(2-(2-Methoxy-ethoxy)-ethyl)-N-methyl-amino)-methyl]-anilin
(191) 4-[N-(N-tert.Butoxycarbonyl-3-amino-propyl)-N-methylaminomethyl]-anilin
(192) 4-[(N-(Methylcarbamoyl-methyl)-N-methyl-amino)-methyl]-anilin
(193) 4-[(N-(Dimethylcarbamoyl-methyl)-N-methyl-amino)-methyl]-anilin
(194) 4-[(N-Propyl-N-methyl-amino)-methyl]-anilin
(195) 4-[(N-(2-Dimethylamino-ethyl)-N-methyl-amino)-methyl]-anilin
(196) 4-[(N-(3-Dimethylamino-propyl)-N-methyl-amino)-methyl]-anilin
(197) 4-[(N-(2-Methoxy-ethyl)-N-methyl-amino)-methyl]-anilin
(198) 4-[(N-(2-Hydroxy-ethyl)-N-methyl-amino)-methyl]-anilin
(199) 4-[(N-(Dioxolan-2-yl-methyl)-N-methyl-amino)-methyl]-anilin
(200) 4-(3-Oxo-piperazin-1-yl-methyl)-anilin
(201) N-[Di-(2-hydroxyethyl)-amino-methylcarbonyl]-N-methyl-p-phenylendiamin
(202) N-[(N-(2-Methoxyethyl)-N-methyl-amino)-methylcarbonyl]-N-methyl-p-phenylendiamin
(203) N-[(N-(2-Dimethylamino-ethyl)-N-methyl-amino)-methylcarbonyl]-N-methyl-p-phenylendiamin
(204) N-[(4-Methyl-piperazin-1-yl)-methylcarbonyl]-N-methyl-p-phenylendiamin
(205) N-[(Imidazol-1-yl)-methylcarbonyl]-N-methyl-p-phenylendiamin
(206) N-[(Phthalimido-2-yl)-methylcarbonyl]-N-methyl-p-phenylendiamin

### Beispiel XXI

### 4-(4-Hydroxymethyl-piperidin-1-yl-methyl-amino)-anilin

1.1 g 4-(4-Ethoxycarbonyl-piperidin-1-yl-methyl-amino)-anilin werden in 15 ml Tetrahydrofuran suspendiert. Bei Raumtemperatur werden 175 mg Lithiumborhydrid zugegeben, 24 h gerührt, erneut 175 mg Lithiumborhydrid zugegeben und nach weiteren 7.5 Stunden 15 mL Wasser zugegeben und 10 Minuten gerührt. Man extrahiert dreimal mit je 15 mL Ethylacetat. Die vereinigten organischen Phasen werden mit Wasser und gesättigter Kochsalzlösung gewaschen, über Natriumsulfat getrocknet und einrotiert. Der Rückstand wird über eine Kieselgel-Säule mit Methylenchlorid/Methanol/Ammoniak 4:1:0.01 als Laufmittel gereinigt.
Ausbeute: 200 mg (27 % der Theorie)
R_{f}-Wert: 0.4 (Kieselgel, Methylenchlorid/Methanol/Ammoniak 4:1:0.01)
Schmelzpunkt: 157°C

### Beispiel XXII

### 4-Methoxycarbonylmethyl-3-nitro-benzoesäuremethylester

54.3 g 3-Nitro-benzoesäuremethylester und 29.0 g Chloressigsäuremethylester werden in 100 ml Dimethylformamid gelöst und diese Lösung wird bei -10°C zu einer Lösung von 78.5 g Kalium-tert.-butylat in 500 ml Diemthylformamid zugetropft. Es wird für weitere 10 Minuten bei Raumtemperatur gerührt und die Lösung nach dieser Zeit auf 350 ml konzentrierte Salzsäure in 2 1 Eiswasser gegossen. Die Lösung wird 0.5 Stunden gerührt, der erhaltene Niederschlag abgesaugt und mit Wasser gewaschen. Das Produkt wird aus 150 ml Methanol umkristallisiert und im Vakuum bei 40°C getrocknet.
Ausbeute: 48.3 g (51 % der Theorie), enthält ca. 20 % 6-Methoxycarbonylmethyl-3-nitro-benzoesäuremethylester
R_{f}-Wert: 0.7 (Kieselgel, Petrolether/Essigester = 1:1)
Schmelzpunkt: 65-73 °C

Analog Beispiel XXII wird folgende Verbindung hergestellt:
(1) 4-Methoxycarbonylmethyl-3-nitro-benzoesäureethylester Hergestellt aus 4-thoxycarbonylmethyl-3-nitro-benzoesäureethylester

### Beispiel XXIII

### 2-Indolinon-6-carbonsäuremethylester

48.3 g 4-Methoxycarbonylmethyl-3-nitro-benzoesäuremethylester werden in 800 ml konzentrierter Essigsäure gelöst, 5.0 g Palladium auf Kohlenstoff (10 prozentig) zugesetzt und die Lösung 2.5 Stunden bei Raumtemperatur und 50 psi hydriert. Der Katalysator wird abfiltriert und das Filtrat eingedampft. Der Rückstand wird in 150 ml tert.-Butylmethylether aufgenommen, erneut filtriert und im Vakuum bei 100°C getrocknet.
Ausbeute: 28.6 g (98 % der Theorie),
R_{f}-Wert: 0.4 (Kieselgel, Methylenchlorid/Methanol = 10:1)
Schmelzpunkt: 208-211 °C

Analog Beispiel XXIII wird folgende Verbindung hergestellt:
(1) 2-Indolinon-6-carbonsäureethylester
   Hergestellt aus 4-Methoxycarbonylmethyl-3-nitro-benzoesäureethylester

### Beispiel XXIV

### 1-Acetyl-3-(1-ethoxy-1-phenylmethylen)-6-ethoxycarbonyl-2-indolinon

15.0 g 2-Indolinon-6-carbonsäureethylester, 49.6 ml Orthobenzoesäuretriethylester und 150 ml Acetanhydrid werden 4 Stunden bei 110°C gerührt. Nach dieser Zeit wird das Lösungsmittel abgezogen, der Rückstand aus Petrolether umkristallisiert und im Vakuum bei 50°C getrocknet.
Ausbeute: 16.9 g (61 % der Theorie),
R_{f}-Wert: 0.5 (Kieselgel, Petrolether/Methylenchlorid/Essigester = 5:4:1)
Schmelzpunkt: 98-100°C
C₂₂H₂₁NO₅

Analog Beispiel XXIV werden folgende Verbindungen hergestellt:
(1) 1-Acetyl-3-(1-ethoxy-1-phenylmethylen)-6-methoxycarbonyl-2-indolinon
   Hergestellt aus 2-Indolinon-6-carbonsäuremethylester, Orthobenzoesäuretriethylester und Acetanhydrid
(2) 1-Acetyl-3-(1-ethoxy-1-ethyl-methylen)-6-ethoxycarbonyl-2-indolinon
   Hergestellt aus 2-Indolinon-6-carbonsäureethylester, Orthopropionsäuretriethylester und Acetanhydrid

### Herstellung der Endverbindungen:

### Beispiel 1

### 3-Z-[1-(4-(Piperidin-1-yl-methyl)-anilino)-1-phenyl-methylen]-6-carbamoyl-2-indolinon-trifluoracetat

300 mg gemäß Beispiel II erhaltenes Harz werden in 3 ml Dimethylformamid suspendiert und mit 0.2 g 4-(Piperidin-1-yl-methyl)-anilin 22 Stunden bei 70°C geschüttelt. Anschließend wird abfiltriert und das Harz mehrmals mit Methylenchlorid, Methanol und Dimethylformamid gewaschen. Dann gibt man für 2 Stunden 1 ml methanolischen Ammoniak zu, um die Acetylgruppe zu entfernen. Anschließend gibt man nach weiterem Waschen 4 ml 10%ige Trifluoressigsäure in Methylenchlorid während 60 Minuten zu, trennt das Harz ab und engt die Lösung ein.
Ausbeute: 69 mg
R_{f}-Wert: 0.1 (Kieselgel, Methylenchlorid/Methanol = 9:1) C₂₈H₂₈N₄O₂
Massenspektrum: m/z = 452 (M⁺)

### Beispiel 2

### 3-Z-[1-(3-(Piperidin-1-yl-methyl)-anilino)-1-phenyl-methylen]-6-carbamoyl-2-indolinon-trifluoracetat

2.0 g gemäß Beispiel II erhaltenem Harz werden analog Beispiel 1 mit 2.0 g 3-Aminobenzylalkohol in 20 ml Dimethylformamid 22 Stunden bei 70°C umgesetzt. Dann wird das Lösungsmittel abgesaugt und das Harz mit Dimethylformamid und Methylenchlorid mehrmals gewaschen. Anschließend werden 200 mg des feuchten beladenen Harzes in 2 ml Methylenchlorid suspendiert und mit 0.2 ml Methansulfonsäurechlorid und 0.1 ml Triethylamin 2 Stunden bei Raumtemperatur stehen lassen. Anschließend wird das Harz mehrmals mit Methylenchlorid gewaschen, in 2 ml Methylenchlorid suspendiert und mit 0.2 ml Piperidin versetzt. Nach 1 Stunde wird das Harz mit Methylenchlorid und Dimethylformamid gewaschen und dann analog Beispiel 1 mit Trifluoressigsäure behandelt.
Ausbeute: 15 mg
R_{f}-wert: 0.30 (Kieselgel, Methylenchlorid/Methanol = 4:1) C₂₈H₂₈N₄O₂
Massenspektrum: m/z = 452 (M⁺)

### Beispiel 3

### 3-Z-[1-(4-(Piperidin-1-yl-methyl)-anilino)-1-phenyl-methylen]-6-ethoxycarbonyl-2-indolinon

1.5 g 1-Acetyl-3-(1-ethoxy-1-phenylmethylen)-6-ethoxycarbonyl-2-indolinon und 1.1 g 4-(Piperidin-1-yl-methyl)-anilin werden in 15 ml Dimethylformamid gelöst und 45 Minuten bei 100°C gerührt. Nach dem Abkühlen werden 5.0 ml Piperidin zugegeben und weitere 3 Stunden bei Raumtemperatur gerührt. Das Lösungsmittel wird abgezogen und der Rückstand über eine AluminiumoxidSäule(Aktivität: 2-3) mit Methylenchlorid/Ethanol (100:3) als Laufmittel aufgereinigt.
Ausbeute: 1.1 g (58% der Theorie),
R_{f}-Wert: 0.5 (Aluminiumoxid, Methylenchlorid/Ethanol = 100:3) C₃₀H₃₁N₃O₃
Massenspektrum: m/z = 481 [M⁺]

### Beispiel 4

### 3-Z-[1-(4-Carboxy-anilino)-1-phenyl-methylen]-6-ethoxycarbon-yl-2-indolinon

485 mg 3-Z-[1-(4-tert.Butoxycarbonyl-anilino)-1-phenyl-methylen]-6-ethoxycarbonyl-2-indolinon werden in 15 ml Methylenchlorid gelöst und 6.0 ml Trifluoessigsäure zugegeben. Das Gemisch wird 2 Stunden bei Raumtemperatur gerührt. Anschließend wird das Lösungsmittel abgezogen und der Rückstand aus Ether umkristallisiert.
Ausbeute: 375 mg (87 % der Theorie),
R_{f}-Wert: 0.3 (Kieselgel, Methylenchlorid/Methanol = 10:1) C₂₅H₂₀N₂O₅
Massenspektrum: m/z = 428 [M⁺]

### Beispiel 5

### 3-Z-[1-(4-Methylaminomethyl-anilino)-1-phenyl-methylen]-6-ethoxycarbonyl-2-indolinon

100 mg 3-Z-[1-(4-(N-Benzyl-N-methyl-aminomethyl)-anilino)-1-phenyl-methylen]-6-ethoxycarbonyl-2-indolinon werden in 20 ml Ethanol gelöst, 0.2 ml 1N Salzsäure zugesetzt und das Gemisch 70 Minuten bei Raumtemperatur und 50 psi Wasserstoffdruck hydriert. Die Reaktionslösung wird filtriert und das Filtrat einrotiert. Der Rückstand wird bei 100°C im Vakuum getrocknet. Ausbeute: 50 mg (53 % der Theorie),
R_{f}-Wert: 0.3 (Kieselgel, Methylenchlorid/Ethanol/Ammoniak = 5:1:0.01)
C₂₆H₂₅N₃O₃
ESI-Massenspektrum: m/z = 426 [M-H⁻]

### beispiel 6

### 3-Z-[1-(4-Ureidomethyl-anilino)-1-phenyl-methylen]-6-methoxycarbonyl-2-indolinon

300 mg 3-Z-[1-(4-Aminomethyl-anilino)-1-phenyl-methylen]-6-methoxycarbonyl-2-indolinon werden in 15 ml Methanol gelöst und 200 ml Triethylamin zugegeben. Anschließend werden 400 mg Kaliumcyanat in 5 ml Wasser zugegeben. Nach 2 Tagen Rühren bei Raumtemperatur wird die Reaktionslösung einrotiert, der Rückstand in Methylenchlorid aufgenommen und je einmal mit Wasser und gesättigter Natriumchloridlösung gewaschen. Die organische Phase wird über Natriumsulfat getrocknet und einrotiert. Der Rückstand wird bei 100°C im Vakuum getrocknet.
Ausbeute: 100 mg (21 % der Theorie),
R_{f}-Wert: 0.7 (Kieselgel, Methylenchlorid/Methanol = 5:1) C₂₅H₂₂N₄O₄
ESI-Massenspektrum: m/z = 441 [M-H⁻]

### Beispiel 7

### 3-Z-[1-(4-Guanidinomethyl-anilino)-1-phenyl-methylen]-6-methoxycarbonyl-2-indolinon

300 mg 3-Z-[1-(4-Aminomethyl-anilino)-1-phenyl-methylen]-6-methoxycarbonyl-2-indolinon werden in 5 ml Dimethylformamid gelöst und 300 ml Triethylamin zugegeben. Anschließend werden 700 mg 3,5-Dimethylpyrazol-1-carbonsöureamidin in 5 ml Dimethylformamid zugegeben. Nach einem Tag Rühren bei Raumtemperatur wird die Reaktionslösung einrotiert. Der Rückstand wird bei 100°C im Vakuum getrocknet.
Ausbeute: 200 mg (87 % der Theorie),
R_{f}-Wert: 0.1 (Reversed Phase RP 8, Methanol/fünfprozentige Kochsalzlösung = 6:4)
C₂₅H₂₃N₅O₃
Massenspektrum: m/z = 441 [M⁺]

### Beispiel 8

### 3-Z-[1-(4-Acetylaminornethyl-anilino)-1-phenyl-methylen]-6-methoxycarbonyl-2-indolinon

100 mg 3-Z-[1-(4-Aminomethyl-anilino)-1-phenyl-methylen]-6-methoxycarbonyl-2-indolinon werden in 5 ml Eisessig gelöst, 0.1 ml Essigsäureanhydrid zugegeben und das Gemisch 10 Minuten bei Raumtemperatur gerührt. Nach dieser Zeit wird die Reaktionslösung auf gesättigte Sodalösung gegossen und viermal mit Methylenchlorid extrahiert. Die vereinigten organischen Phasen werden mit gesättigter Kochsalzlösung gewaschen, über Natriumsulfat getrocknet und einrotiert. Der Rückstand wird bei 100°C im Vakuum getrocknet.
Ausbeute: 20 mg (23 % der Theorie),
R_{f}-Wert: 0.4 (Kieselgel, Methylenchlorid/Methanol = 10:1) C₂₆H₂₃N₃O₄
ESI-Massenspektrum: m/z = 440 [M-H⁻]

### Beispiel 9

### 3-Z-[1-(4-(Piperidin-1-yl-methyl)-anilino)-1-phenyl-methylen]-6-carboxy-2-indolinon

0.8 g 3-Z-[1-(4-(Piperidin-1-yl-methyl)-anilino)-1-phenyl-methylen]-6-ethoxycarbonyl-2-indolinon werden in 30 ml Ethanol gelöst, 8.3 ml 1N Natronlauge zugegeben und die Mischung 1 Stunde bei 80°C gerührt. Nach dem Abkühlen wird mit 8.3 ml 1N Salzsäure neutralisiert. Der ausgefallene Niederschlag wird abgesaugt, mit Wasser, Ethanol und Ether gewaschen und im Vakuum bei 100°C getrocknet.
Ausbeute: 0.7 g (89 % der Theorie),
R_{f}-Wert: 0.2 (Kieselgel, Methylenchlorid/Methanol = 5:2) C₂₈H₂₇N₃O₃
Massenspektrum: m/z = 453 [M⁺]

### Beispiel 10

### 3-Z-[1-(4-(Piperidin-1-yl-methyl)-anilino)-1-phenyl-methylen]-6-methoxycarbonyl-2-indolinon

0.9 g 3-Z-[1-(4-(Piperidin-1-yl-methyl)-anilino)-1-phenyl-methylen]-6-carboxy-2-indolinon werden in 35 ml Dimethylformamid suspendiert und 0.4 g Carbonyldiimidazol zugegeben. Das Gemisch wird 14 Stunden bei 80°C gerührt. Nach dieser Zeit werden 20 ml Methanol zugegeben und nochmals 3 Stunden bei - 50°C gerührt. Das Lösunsmittel wird abgezogen und der Rückstand über eine Kieselgelsäule mit Methylenchlorid/Methanol (3:1) als Laufmittel aufgereinigt.
Ausbeute: 0.5 g (49% der Theorie),
R_{f}-Wert: 0.5 (Aluminiumoxid, Methylenchlorid/Methanol = 30:1) C₂₉H₂₉N₃O₃
ESI-Massenspektrum: m/z = 468 [M+H⁺]

### Beispiel 11

### 3-Z-[1-(4-(Piperidin-1-yl-methyl)-anilino)-1-phenyl-methylen]-6-carbamoyl-2-indolinon

0.9 g 3-Z-[1-(4-(Piperidin-1-yl-methyl)-anilino)-1-phenyl-methylen]-6-carboxy-2-indolinon, 0.8 g TBTU und 0.4 g HOBT werden in 25 ml Dimethylformamid suspendiert und 1.0 ml Triethylamin zugegeben. Die Mischung wird 15 Minuten bei Raumtemperatur gerührt. Nach dieser Zeit wird bei 10-15°C über 15 Minuten Ammoniakgas eingeleitet und 1.5 Stunden bei Raumtemperatur gerührt. Der entstandene Niederschlag wird abgesaugt, mit Wasser, Ethanol und Ether gewaschen und im Vakuum bei 100°C getrocknet.
Ausbeute: 0.6 g (64 % der Theorie),
R_{f}-Wert: 0.4 (Reversed Phase RP 8, Methanol/fünfprozentige Kochsalzlösung = 6:4)
C₂₈H₂₈N₄O₂
ESI-Massenspektrum: m/z = 453 [M+H⁺]

### Beispiel 12

### 3-Z-[1-(4-(N-Dimethylaminomethylcarbonyl-N-methyl-amino)-anilino)-1-phenyl-methylen]-6-methoxycarbonyl-2-indolinon x Citronensäure

3.25 g Citronensäuremonohydrat werden in 50 ml Methanol vorgelegt und 5.0 g 3-Z-[1-(4-(N-Dimethylaminomethylcarbonyl-N-methyl-amino)-anilino)-1-phenyl-methylen]-6-methoxycarbonyl-2-indolinon bei Raumtemperatur zugegeben. Die entstandene Lösung wird eingeengt, der Rückstand mit Ether gewaschen und aus Ethylacetat umkristallisiert.
Ausbeute: 6.3 g (90 % der Theorie),
R_{f}-Wert: 0.6 (Kieselgel, Methylenchlorid/Methanol/Ammoniak = 5:1:0.01)
Schmelzpunkt: 198°C
C₂₈H₂₈N₄O5 x C₆H₈O₇
ESI-Massenspektrum: m/z = 483 [M-H⁻]

| | | | | |
|---|---|---|---|---|
| Elementaränalyse: | Ber.: | C 60.34 | H 5.37 | N 8.28 |
| | Gef.: | 59.98 | 5.25 | 8.13 |

Analog den vorstehenden Beispielen kann die erfindungsgmäße Verbindung hergestellt werden:
(473) 3-Z-[1-(4-(N-((4-Methyl-piperazin-1-yl)-methylcarbonyl)-N-methyl-amino)-anilino)-1-phenyl-methylenl-6-methoxycarbonyl-2-indolinon

### Beispiel 13

### Trockenampulle mit 75 mg Wirkstoff pro 10 ml

### Zusammensetzung:

| | |
|---|---|
| Wirkstoff | 75.0 mg |
| Mannitol | 50.0 mg |
| Wasser für Injektionszwecke | ad 10.0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel 14

### Trockenampulle mit 35 mg Wirkstoff pro 2 ml

### Zusammensetzung:

| | |
|---|---|
| Wirkstoff | 35.0 mg |
| Mannitol | 100.0 mg |
| Wasser für Injektionszwecke | ad 2.0 ml |

### Herstellung:

Wirkstoff und Mannitol werden in Wasser gelöst. Nach Abfüllung wird gefriergetrocknet.

Die Auflösung zur gebrauchsfertigen Lösung erfolgt mit Wasser für Injektionszwecke.

### Beispiel 15

### Tablette mit 50 mg Wirkstoff

### Zusammensetzung:

| | | |
|---|---|---|
| (1) | Wirkstoff | 50.0 mg |
| (2) | Milchzucker | 98.0 mg |
| (3) | Maisstärke | 50.0 mg |
| (4) | Polyvinylpyrrolidon | 15.0 mg |
| (5) | Magnesiumstearat | 2.0 mg |
| | | 215.0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

### Beispiel 16

### Tablette mit 350 mg Wirkstoff

### Zusammensetzung:

| | | |
|---|---|---|
| (1) | Wirkstoff | 350.0 mg |
| (2) | Milchzucker | 136.0 mg |
| (3) | Maisstärke | 80.0 mg |
| (4) | Polyvinylpyrrolidon | 30.0 mg |
| (5) | Magnesiumstearat | 4.0 mg |
| | | 600.0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

### Beispiel 17

### Kapseln mit 50 mg Wirkstoff

### Zusammensetzung:

| | | |
|---|---|---|
| (1) | Wirkstoff | 50.0 mg |
| (2) | Maisstärke getrocknet | 58.0 mg |
| (3) | Milchzucker pulverisiert | 50.0 mg |
| (4) | Magnesiumstearat | 2.0 mg |
| | | 160.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

### Beispiel 18

### Kapseln mit 350 mg Wirkstoff

### Zusammensetzung:

| | | |
|---|---|---|
| (1) | Wirkstoff | 350.0 mg |
| (2) | Maisstärke getrocknet | 46.0 mg |
| (3) | Milchzucker pulverisiert | 30.0 mg |
| (4) | Magnesiumstearat | 4.0 mg |
| | | 430.0 mg |

### Herstellung;

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

### Beispiel 19

### Suppositorien mit 100 mg Wirkstoff

### 1 Zäpfchen enthält:

| | | |
|---|---|---|
| | Wirkstoff | 100.0 mg |
| | Polyethylenglykol (M.G. 1500) | 600.0 mg |
| | Polyethylenglykol (M.G. 6000) | 460.0 mg |
| | Polyethylensorbitanmonostearat | 840.0 mg |
| | | 2 000.0 mg |

### Herstellung:

Das Polyethylenglykol wird zusammen mit Polyethylensorbitanmonostearat geschmolzen. Bei 40°C wird die gemahlene Wirksubstanz in der Schmelze homogen dispergiert. Es wird auf 38°C abgekühlt und in schwach vorgekühlte Suppositorienformen ausgegossen.

## Patentansprüche

1. 3-Z-[1-(4-(N-((4-Methyl-piperazin-1-yl)-methylcarbonyl)-N-methyl-amino)-anilino)-l-phenylmethylene]-6-methoxycarbonyl-2-indolinon, dessen Tautomere und dessen Salze.

2. Physiologisch verträgliche Salze der Verbindung gemäß Anspruch 1.

3. Arzneimittel, enthaltend eine Verbindung nach Anspruch 1 oder ein Salz gemäß Anspruch 2 neben gegebenenfalls einem oder mehreren inerten Trägerstoffen und/oder Verdünnungsmitteln.

4. Verbindung nach Anspruch 1 oder Salz gemäß Anspruch 2 zur Verwendung als Arzneimittel.

5. Verbindung nach Anspruch 1 oder Salz gemäß Anspruch 2 zur Verwendung zur Behandlung von exzessiven oder anormalen Zellproliferationen.

6. Verwendung einer Verbindung nach Anspruch 1 oder eines Salzes gemäß Anspruch 2 zur Herstellung eines Arzneimittels, welches zur Behandlung von exzessiven oder anormalen Zellproliferationen geeignet ist.

7. Verfahren zur Herstellung eines Arzneimittels gemäß Anspruch 3, **dadurch gekennzeichnet, daß** auf nichtchemischem Wege eine Verbindung nach Anspruch 1 oder ein Salz gemäß Anspruch 2 in einen oder mehrere inerte Trägerstoffe und/oder Verdünnungsmittel eingearbeitet wird.

## Claims

1. 3-Z-[1-(4-N-((4-Methyl-piperazin-1-yl)-methylcarbonyl)-N-methyl-amino)-anilino)-1-phenyl-methylene]-6-methoxycarbonyl-2-indolinone, the tautomers and the salts thereof.

2. Physiologically acceptable salts of the compound according to claim 1.

3. Medicament containing a compound according to claim 1 or a salt according to claim 2, optionally together with one or more inert carriers and/or diluents.

4. Compound according to claim 1 or salt according to claim 2 for use as a medicament.

5. Compound according to claim 1 or salt according to claim 2 for use in the treatment of excessive or abnormal cell proliferation.

6. Use of a compound according to claim 1 or a salt according to claim 2 for preparing a medicament which is suitable for treating excessive or abnormal cell proliferation.

7. Process for preparing a medicament according to claim 3, **characterised in that** a compound according to claim 1 or a salt according to claim 2 is incorporated in one or more inert carriers and/or diluents by a non-chemical method.

## Revendications

1. 3-Z-[1-(4-(N-((4-méthyl-pipérazin-1-yl)-méthylcarbonyl)-N-méthyl-amino)-anilino)-1-phényl-méthylène]-6-méthoxycarbonyl-2-indolinone, ses tautomères et ses sels.

2. Sels physiologiquement acceptables du composé selon la revendication 1.

3. Médicament contenant un composé selon la revendication 1 ou un sel selon la revendication 2, outre éventuellement un ou plusieurs véhicules et/ou diluants inertes.

4. Composé selon la revendication 1 ou sel selon la revendication 2 pour l'utilisation comme médicament.

5. Composé selon la revendication 1 ou sel selon la revendication 2, destiné à l'utilisation pour le traitement des proliférations cellulaires excessives ou anormales.

6. Utilisation d'un composé selon la revendication 1 ou d'un sel selon la revendication 2 pour la production d'un médicament qui est approprié pour le traitement des prolifération cellulaires excessives ou anormales,

7. Procédé de production d'un médicament selon la revendication 3, **caractérisé en ce qu'**un composé selon la revendication 1 ou un sel selon la revendication 2 est intégré de façon non chimique dans un ou plusieurs véhicules et/ou diluants inertes.
